(19) Europäisches Patentamt

European Patent Office

Office européen des brevets

(11) **EP 1 368 052 B1**

(12) **EUROPÄISCHE PATENTSCHRIFT**

(45) Veröffentlichungstag und Bekanntmachung des Hinweises auf die Patenterteilung:
**10.08.2005 Patentblatt 2005/32**

(21) Anmeldenummer: 02761890.9

(22) Anmeldetag: **21.02.2002**

(51) Int Cl.⁷: **A61K 38/08**, A61P 17/02

(86) Internationale Anmeldenummer:
**PCT/EP2002/001828**

(87) Internationale Veröffentlichungsnummer:
**WO 2002/083160 (24.10.2002 Gazette 2002/43)**

(54) **MITTEL, DIE DIE APOPTOSE BEI AN DER WUNDHEILUNG BETEILIGTEN ZELLEN INHIBIEREN**

AGENTS, WHICH INHIBIT APOPTOSIS IN CELLS THAT ARE INVOLVED IN WOUND HEALING

AGENTS INHIBANT L'APOPTOSE DANS DES CELLULES PARTICIPANT A LA CICATRISATION

(84) Benannte Vertragsstaaten:
**AT BE CH CY DE DK ES FI FR GB GR IE IT LI LU MC NL PT SE TR**

(30) Priorität: **26.02.2001 DE 10109136**

(43) Veröffentlichungstag der Anmeldung:
**10.12.2003 Patentblatt 2003/50**

(73) Patentinhaber: **Dermatools Biotech GmbH 64287 Darmstadt (DE)**

(72) Erfinder:
- **FREYBERG, Mark, Andre 64287 Darmstadt (DE)**
- **FRIEDL, Peter 64823 Gross-Umstadt (DE)**
- **KAISER, Dirk 64859 Eppertshausen (DE)**

(74) Vertreter: **Luderschmidt, Schüler & Partner GbR Patentanwälte Industriepark Höchst, Gebäude F 821 65926 Frankfurt (DE)**

(56) Entgegenhaltungen:
**EP-A- 1 005 873          WO-A-99/43787
DE-A- 19 952 960          US-A- 5 627 265**

- **FREYBERG M A ET AL: "Integrin-associated protein and thrombospondin-1 as endothelial mechanosensitive death mediators." BIOCHEMICAL AND BIOPHYSICAL RESEARCH COMMUNICATIONS. UNITED STATES 19 MAY 2000, Bd. 271, Nr. 3, 19. Mai 2000 (2000-05-19), Seiten 584-588, XP001120601 ISSN: 0006-291X**

EP 1 368 052 B1

## Beschreibung

Die vorliegende Erfindung betrifft Mittel, die an der Wundheilung beteiligte Zellen anti-apoptotisch beeinflussen. Insbesondere betrifft die vorliegende Erfindung die Verwendung von Substanzen, die entweder an

IAP und/oder an Integrin $\alpha_v\beta_3$ und/oder an Thrombospondin 1 so binden, daß die Bindung zwischen Thrombospondin 1 und IAP und/oder Integrin $\alpha_v\beta_3$ inhibiert wird, wodurch die Apoptoserate bei mit der Wundheilung assoziierten Zellen verringert wird, zur Herstellung von Medikamenten, die sich zur Behandlung von Wunderkrankungen, bevorzugt chronischen Wunden, eignen. Bevorzugte Ausführungsbeispiele umfassen die in den SEQ ID Nos 1-11 dargestellten Aminosäuresequenzen bzw. daraus abgeleitete Peptidmimetika. Weiterhin betrifft die vorliegende Erfindung Zellen mit signifikant verringerter Apoptoserate, wobei diese Zellen bevorzugt als Bestandteil einer an sich im Stand der Technik bereits beschriebenen, sogenannten künstlichen Haut verwendet werden können, und wobei vor Anlegen dieser Haut auf die Wunde die Haut mechanisch gespannt wird, und wobei die entsprechenden Zellen bevorzugt zusätzlich mit den erfindungsgemäßen Mitteln behandelt werden.

Der Prozess der Wundheilung besteht aus drei Phasen, während derer das verleizte Gewebe repariert wird und sich somit regeneriert, wonach das neue Gewebe sich als Narbe manifestiert.

Diese drei Phasen werden folgendermaßen klassifiziert:

(a) Entzündungsphase, die nach 0 bis 3 Tagen beginnt

(b) eine sich anschließende zelluläre Proüferationsphase von 3 bis 12 Tagen,und

(c) eine Aufbauphase von 3 Tagen bis etwa 6 Monaten.

In der Entzündungsphase versammeln sich Entzündungszellen, hauptsächlich Neutrophile, an der Wundstelle, gefolgt von Lymphozyten, Monozyten und noch später Makrophagen.

Während der Proliferationsphase wird das sogenannte Granulationsgewebe (bei der Ausheilung von Wunden, Geschwüren u.a. sich bildendes gefäßreiches Bindegewebe) im verletzten Gebiet gebildet. Hierbei spielen neben anderen Zelltypen vor allem Fibroblasten und Epithelzellen (Re-Epithelisierung) eine wichtige Rolle. Die Fibroblasten produzieren das für die Wundheilung wichtige Kollagen.

Für die Bildung von Kollagen ist Ascorbinsäure (Vitamin C) essentiell. Es wurde schon in einigen Untersuchungen gezeigt, daß die Anwendung von Ascorbinsäure die reduzierte proliferative Aktivität und die Kollagen-Synthese-Aktivität von gealterten Hautfibroblasten aktiviert, und daher die Wundheilung verbessert. Die Wirkungsweise ist allerdings nicht gezeigt worden, es wird jedoch u.a. eine Beteiligung von Vitamin C an Lipid-Oxidationsvorgängen beschrieben.

Bei der Re-Epithelisierung proliferieren die Ephitelzellen und wandern von den Wundrändem her in das Gewebe ein. Hier konnte gezeigt werden, daß die Re-Epithelisierung durch Wundverbände, die eine Feuchtigkeitsbarriere darstellen, gefördert werden kann.

Die Schlußphase der Wundheilung ist gekennzeichnet durch das Ersetzen des Granulationsgewebes durch Kollagen und Elastin-Fasern und die Devaskularisierung des Granulationsgewebes (d.h. Bildung von Narbengewebe). Jüngste Studien haben dabei gezeigt, daß die topische Anwendung von Anti-Oxidantien wie alpha-Tocopherol die Narbenbildung verringert und die Blutgerinnung während der Therapie normalisiert.

Gerade Fibroblasten und Epithelzellen spielen daher eine wichtige Rolle bei der Wundheilung. Dabei steht die geringe Proliferationsaktivität der Fibroblasten und deren geringe Anzahl einer günstigen Wundheilung entgegen. Vielfach werden neben entzündungshemmenden Stoffen auch Stoffe wie Wachstumsfaktoren eingesetzt, die die Proliferationsfähigkeiten oder die Syntheseaktivität der beteiligten Zellen verstärken sollen.

Die Apoptose (Synonym: programmierter Zelltod) ist ein unumkehrbarer Prozeß. Eine apoptotische Zelle stirbt unweigerlich ab. Eine negative Rolle apoptotischer Fibroblasten bei der Wundheilung wird diskutiert, es wurde jedoch noch kein Weg vorgeschlagen, wie die Apoptoserate der Fibroblasten bei der Wundheilung günstig beeinflusst werden kann, indem bestimmte Medikamente, also molekulare Heilmittel mit den Fibroblasten in Kontakt gebracht werden.

In der europäischen Patentanmeldung EP-A-0 903 149 wird ein Verfahren zur Identifizierung Apoptose-auslösender Substanzen bei Immunzellen beschrieben. Es wurde gezeigt, daß Substanzen, die an das Integrinassoziierte Protein (IAP bzw. CD 47) auf der Oberfläche von Inmunzellen binden, die Fähigkeit besitzen können, Apoptose auszulösen. Die Wirkmechanismen wurden nicht beschrieben.

Es wurde bereits vorgeschlagen, daß IAP an der Bildung eines spezifischen Calciumkanals beteiligt ist (Schwartz, M. A. *et al.*, Journal of Biological Chemistry, 268:27, 19931-19934). Eine Rolle dieses hypothetischen Calcium-Kanals bei der Auslösung der Apoptose wurde nicht erwähnt.

In der prioritätsälteren Internationalen Patentanmeldung WO 01/33218 A1 der Anmelderin, auf die für die Zwecke der vorliegenden Anmeldung vollinhaltlich Bezug genommen wird, wird ein Verfahren zur Identifizierung Apoptose-inhibierender Substanzen und die Verwendung solcher Substanzen zur Herstellung von Medikamenten zur Behandlung von Gefäßerkrankungen bzw. als aktives Prinzip einer pharmazeutischen Zubereitung zur Behandlung von Gefäßerkrankungen beschrieben. Eine Verwendung solcher Substanzen zur Herstellung von Medikamenten zur Förderung der Wundheilung bzw. als aktives Prinzip einer pharmazeutischen Zubereitung zur Behandlung von Wunden wurde nicht beschrieben.

Einer der modernsten Ansätze zur Behandlung chronischer Wunden ist die Verwendung einer sogenannten lebenden Haut (living skin) als Hautersatz oder Wundenabdeckmittel. Es gibt bereits eine Vielzahl von Patentanmeldungen auf diesem Gebiet. Stellvertretend sei hier die EP 1 005 873 der Isotis N.V. oder die Internationale Patenanmeldung WO 99/43787 der Advanced Tissue Sciences Inc. genannt. Prinzipiell werden zur Herstellung einer solchen künstlichen Haut entweder autologe oder heterologe Zellen (Hautzellen) auf einer biokompatiblen Membran gezüchtet und diese künstliche Haut wird dann als Heilmittel eingesetzt. Es wurde bisher jedoch nicht beschrieben, daß es günstig oder notwendig sei, die Apoptose bei den verwendeten Zellen zu hemmen, um einen günstigeren Verlauf der Heilung zu gewährleisten. Weder ein Verstrecken der künstlichen Haut noch der Zusatz von anti-apoptotischen Mitteln wird vorgeschlagen. Ein negativer Einfluß von Apoptose auf den Heilungsverlauf wird in diesen Patentanmeldungen nicht diskutiert und dem Fachmann nicht nahegelegt.

Gerade chronische Wunderkrankungen oder großflächige und/oder tiefgehende Wunden, wie sie bei Brandverletzungen häufig auftreten, lassen sich zur Zeit trotz des genannten Stands der Technik nur schwer heilend beeinflussen.

Es besteht daher im Stand der Technik noch immer ein hoher Bedarf an verbesserten Mitteln, die die Wundheilung, und hier besonders auch die Heilung chronischer Wunden oder großflächiger Wunden bzw. Brandverletzungen der Haut, günstig beeinflussen.

Der vorliegenden Erfindung liegt daher die Aufgabe zugrunde, Stoffe und/oder Mittel zur Verfügung zu stellen, die für die Herstellung eines Medikamentes bzw. eines Mittels zur Behandlung von Wunderkrankungen und/oder Brandverletzungen geeignet sind.

Gelöst wird diese sowie weitere, nicht explizit genannte Aufgaben, die jedoch aus den hierin einleitend diskutierten Zusammenhängen ohne weiteres ableitbar oder erschließbar sind, durch eine Verwendung nach Patentanspruch 1. Zweckmäßige Abwandlungen der erfindungsgemäßen Verwendung werden in den auf Anspruch 1 rückbezogenen Unteransprüchen unter Schutz gestellt.

Insbesondere wird die Aufgabe gelöst durch die Verwendung von

Substanzen, die entweder an IAP und/oder an Integrin $\alpha_v\beta_3$ und/oder an Thrombospondin 1 so binden, daß die Bindung zwischen Thrombospondin 1 und IAP und/oder Integrin $\alpha_v\beta_3$ inhibiert wird, und die die Apoptoserate bei mit der Wundheilung assoziierten Zellen verringern, zur Herstellung von Medikamenten, die als aktiven Bestandteil mindestens eine solche Substanz enthalten, und die für die Behandlung von Wunden einsetzbar sind, besonders für die Behandlung von chronischen Wunden und/oder Brandverletzungen.

Ein Medikament zur Behandlung der genannten Wundverletzungen kann hergestellt werden, indem zunächst ein Identifikationsverfahren der Stufen (i) bis (v) durchgeführt wird, wobei man:

(i) Zellen züchtet, die sowohl IAP als auch das Integrin $\alpha_v\beta_3$ exprimieren,
(ii) die Zellen zur Bildung einer Apoptose-induzierenden
  Substanz veranlaßt, und/oder eine Substanz bzw.
  Substanzen zusetzt, die Apoptose induziert/induzieren,
(iii) eine Testsubstanz nach einem der Ansprüche 5 bis 9 zusetzt,
(iv) die Apoptoserate misst, und
(v) Testsubstanzen, die eine verringerte Apotoserate hervorbringen, auswählt, und dann
(vi) die so identifizierten Testsubstanzen (Identifikate) mit einem pharmazeutisch geeigneten Träger mischt.

Besonders bevorzugt inhibieren die erfindungsgemäß verwendbaren Substanzen (Identifikate) die Apoptose bei Fibroblasten und/oder Epithelzellen.

Weiterhin bevorzugt umfasst die vorliegende Erfindung Medikamente, die als aktiven Bestandteil eine solche Substanz sowie einen oder mehrere, gegebenenfalls unterschiedliche Fibroblastenwachstumsfaktoren enthalten, wie z. B. den basischen Fibroblastenwachstumsfaktor (bFGF), und die für die Behandlung von Wunden einsetzbar sind, besonders für die Behandlung von chronischen Wunden und/oder Brandverletzungen.

Die Erfindung umfaßt weiterhin bevorzugt die Verwendung dieser Substanzen zur Behandlung von Wunderkrankungen, insbesondere zur Behandlung von chronischen Wunden.

Insbesondere wird die erfindungsgemäße Aufgabe gelöst durch zur Verfügungstellen von Substanzen, die Peptide mit einer der in den SEQ ID NOs 1 bis 11 dargestellten Aminosäuresequenzen umfassen.

Unter einem bevorzugten Ausführungsbeispiel der vorliegenden Erfindung handelt es sich dabei um Apoptose-inhibierende Substanzen, die Aminosäuresequenzen der allgemeinen Formel (1):

R-A1-Y-V-V-M umfassen,

wobei A1 für A, D, E, G, M, N, T, W oder Y steht, oder pharmazeutisch annehmbare Salze dieser Substanzen.

Für die Zwecke der vorliegenden Erfindung wird der international übliche Einbuchstabencode für Aminosäuren verwendet, A steht also für Alanin (Ala), C für Cystein (Cys), D für Asparaginsäure (Asp), E für Glutaminsäure (Glu), F für Phenylalanin (Phe), G für Glycin (Gly), L für Leucin (Leu), M für Methionin (Met), N für Asparagin (Asn), P für Prolin (Pro), R für Arginin (Arg), S für Serin (Ser), T für Threonin (Thr), V für Valin (Val), W für Tryptophan (Trp) und Y für Tyrosin (Tyr).

Unter einem besonders bevorzugten Gesichtspunkt betrifft die vorliegende Erfindung daher Apoptose inhibierende Substanzen, bevorzugt Proteine oder Peptide, die eine der unter SEQ ID NO 1 bis SEQ ID NO 11 gezeigten Peptidsequenzen umfassen, oder deren entsprechende pharmazeutisch annehmbare Salze.

Weiterhin betrifft die vorliegende Erfindung die Verwendung der erfindungsgemäßen Substanzen, bevorzugt Proteine oder Peptide umfassend mindestens eine der in SEQ ID NO 1 bis SEQ ID NO 11 dargestellten Aminosäuresequenz zur Herstellung von Medikamenten, insbesondere zur Herstellung von Medikamenten zur Behandlung von Wunderkrankungen, und ganz besonders bevorzugt hierbei chronische Wunden oder schwere Brandverletzungen.

Überraschenderweise wurde von den Erfindern gezeigt, daß Peptide die eine durch Formel (1) dargestellte Aminosäuresequenz umfassen, in überaus starkem Maße Apoptose inhibieren. Insbesondere sind diese Peptide hervorragend dazu geeignet, die TSP-1 induzierte Apoptose zu inhibieren.

Als ganz besonders bevorzugte Ausführungsbeispiele der vorliegenden Erfindung werden folgende Peptide/Peptidsequenzen zur Verfügung gestellt:

1. R-A-Y-V-V-M (SEQ ID NO 1)

2. R-W-Y-V-V-M (SEQ ID NO 2)

3. R-Y-Y-V-V-M (SEQ ID NO 3)

4. R-E-Y-V-V-M (SEQ ID NO 4)

5. K-R-A-Y-V-V-M-W-K-K (SEQ ID NO 5)

6. K-R-E-Y-V-V-M-W-K-K (SEQ ID NO 6)

7. R-G-Y-V-V-M (SEQ ID NO 7)

8. R-M-Y-V-V-M (SEQ ID NO 8)

9. R-T-Y-V-V-M (SEQ ID NO 9)

10. R-N-Y-V-V-M (SEQ ID NO 10)

11. R-D-Y-V-V-M (SEQ ID NO 11)

In einer weiteren bevorzugten Ausführungsform der vorliegenden Erfindung werden an der Wundheilung beteiligte Zellen, bevorzugt Fibroblasten oder Epithelzellen, als eine an sich im Stand der Technik bereits bekannte, sogenannte künstliche Haut kultiviert, und diese wird erfindungsgemäß als Wundheilungsmittel verwendet, wobei diese künstliche Haut nach Anwachsen der Zellen mechanisch gespannt wird und/oder wobei die entsprechenden Zellen vor oder nach dem Aufbringen der künstlichen Haut auf den Ort der Wunde mit den erfindungsgemäßen Mitteln behandelt werden, und wobei der Anteil apoptotischer Zellen an der Gesamtzahl der Zellen um mindestens 10%, bevorzugt um mindestens 50%, besonders bevorzugt um mindestens 75% und ganz besonders bevorzugt um mindestens 90%, verglichen mit einer nicht mechanisch gespannten und mit den erfindungsgemäßen Mitteln nicht behandelten künstlichen Haut, gesenkt werden kann.

Hierbei wird diese Haut bevorzugt nach Anwachsen der Zellen auf der biokompatiblen Membran oder direkt vor der Applikation der Haut auf die Wunde um 10 - 90 % verstreckt, und das erfindungsgemäße Mittel wird bevorzugt 8-24 h nach dem Verstrecken appliziert.

Überraschenderweise wurde von den vorstehend genannten Erfindern gezeigt, daß die Bindung von Thrombospondin-1 (TSP-1) an IAP und/oder das Integrin $\alpha_v\beta_3$ bei Fibroblasten Apoptose auslöst (Beispiele 6 und 7). Die entsprechenden Untersuchungen wurden an MRC-5-Fibroblasten durchgeführt (ATCC-Nr.: CCL-171). Die Apoptoserate wird wie in Beispiel 3 gezeigt bestimmt.

Weiterhin konnte überraschenderweise gezeigt werden, daß das TSP-1 von den Fibroblasten selbst gebildet wird, es sich also um eine selbst-induzierte oder spontane Apoptose handelt (Beispiel 8 und 9). Diese Untersuchungen wurden in herkömmlichen, statischen Zellkulturen durchgeführt. Diese zeichnen sich durch das Fehlen von Strömungen im Zellkulturmedium aus. Es konnte aber unerwarteterweise gezeigt werden, daß von Fibroblasten in einer dynamischen Zellkultur (Kultivierung in der Plattenkegelscherungsapparatur, siehe Beispiel 1), d.h. unter Bedingungen, bei denen die Zellen mit einem strömenden Zellkulturmedium konfrontiert sind, TSP-1 nicht gebildet wird und Apoptose in dieser Zellkultur nicht oder nur in sehr geringem Maße auftritt (Beispiel 5 und 8). Dies war auch deswegen in hohem Maße überraschend, da nicht davon ausgegangen werden kann, daß Fibroblasten unter natürlichen Bedingungen Scherkräften ausgesetzt sind, die durch Flüssigkeitsströmungen hervorgerufen werden.

Eine Supplementation von frischem Medium mit TSP-1 verursacht eine Erhöhung der spontanen Apoptose bei statisch kultivierten Fibroblasten (Beispiel 6). Diese Erhöhung entspricht in etwa dem Effekt von statisch konditioniertem Medium (Beispiel 5). Der Begriff konditioniertes Medium bedeutet hier ein Zellkulturmedium, das vorher bereits zur Kultivierung anderer Zellen verwendet wurde. Dieses Medium zeichnet sich dadurch aus, daß in ihm lösliche Mediatoren, z.B. Wachstumsfaktoren, Hormone, etc. gelöst sind, welche von Zellen im Laufe ihrer Kultivierung gebildet werden. Dieses Ergebnis zeigt, daß statisch konditioniertes Medium die Fähigkeit besitzt, Apoptose über einen Mediator wie TSP-1 zu induzieren. Die Herstellung von konditioniertem Medium ist in Beispiel 2 erläutert.

Durch den Einsatz eines anti-TSP-1-Antikörpers, welcher an TSP-1 bindet und auf diese Art und Weise neutralisiert, konnte gezeigt werden, daß TSP-1 der Mediator der Apoptose von Fibroblasten ist (Beispiel 6). Der Effekt von zugegebenem TSP-1 kann ebenso wie der Effekt von statisch konditioniertem Medium durch ein zugesetztes polyklonales Antiserum gegen TSP-1 wie auch durch den Zusatz eines monoklonalen anti-TSP-1-Antikörpers unterdrückt werden (Beispiel 6).

Die TSP-1 Sekretionsraten wurden für dynamische und statische postkonfluente Kulturen bestimmt (Beispiel 8 und 9).

Der Begriff "statische Zellkultur (-bedingungen)" bedeutet hier eine Kultivierung von Zellen unter Bedingungen, bei denen stetige, d.h. gleichbleibend gerichtete, laminare Strömungen in dem die Zellen umgebenden Zellkulturmedium nicht auftreten. Zu den "statischen Zellkulturbedingungen" im hier verwendeten Sinne zählen also auch solche Zellkulturbedingungen, unter denen turbulente oder unstetige laminare Strömungen, also z.B. solche mit wechselnden Strömungsrichtungen oder gar mit Strömungsumkehr, auftreten. Der Begriff "dynamische Zellkultur (-bedingungen)" bedeutet hier solche Zellkulturbedingungen, bei denen nur gleichbleibend gerichtete, laminare Strömungsverhältnisse im Zellkulturmedium vorherrschen, d.h. solche Zellkulturbedingungen, wie sie im Stand der Technik z.B. mit Hilfe einer Kultivierung in einer Plattenkegelscherungsapparatur erreicht werden können (siehe Beispiel 1). Es ist klar, daß bei Fibroblasten in der in-vivo-Situation Flüssigkeitsströmungen nicht auftreten, da das Bindegewebe, in dem die Fibroblasten vor allem lokalisiert sind, kein flüssigkeitsgefülltes Kompartiment darstellt

In der Zellkultur wirken je nach Strömungsverhältnissen unterschiedliche Scherkräfte auf die kultivierten Zellen ein. Eine gleichbleibend gerichtete laminare Strömung resultiert dabei in einer Scherkraft, die größer als 0,001 dyn/cm$^2$ ist und deren Vektorsumme größer ist als unter unstetigen Strömungsverhältnissen mit wechselnden Strömungsrichtungen. Statische Zellkultur (-bedingungen) zeichnen sich durch deutlich kleinere (< 0,001 dyn/ cm$^2$) bzw. gar keine Krafteinwirkung aus. Dynamische Zellkulturen weisen Scherkräfte von > 0,001 dyn/cm$^2$ bzw. eine Reynoldszahl von > 0,1 auf. Bei Reynoldszahlen von > 200 (-1000) können turbulente Strömungen auftreten (abhängig von der Geometrie der Strömungskammer), die wie statische oder unstetige Strömungsbedingungen keinen protektiven Charakter mehr auf die Auslösung der Apoptose haben. Für die Untersuchungen, die zur vorliegenden Erfindung führten, wurden verschiedene Kultivierungsmethoden benutzt. Diese sind in Beispiel 1 beschrieben.

Die Zugabe von TSP-1 zu einer dynamischen Fibroblastenzellkultur hat überraschenderweise im Gegensatz zur statischen Kultur keinen Effekt auf die Apoptose (Beispiel 5). Dies zeigt, daß die Apoptose nicht nur von der Produktion von TSP-1, sondern vielmehr auch vom Auftreten bzw. der Zugänglichkeit spezifischer Rezeptoren auf der Oberfläche der Zellen abhängt. Hierbei konnte überraschenderweise gezeigt werden, daß der Rezeptor Integrin $\alpha_v\beta_3$ auf statisch und dynamisch kultivierten Zellen nachzuweisen ist, wohingegen der Rezeptor IAP nur in statischer Kultur in nachweisbaren Mengen exprimiert wird.

Es ist bekannt, daß TSP-1 an das Integrin $\alpha_v\beta_3$ bindet. Die Bindung von TSP-1 an das $\alpha_v\beta_3$-Integrin wird über ein RGD-Sequenzmotiv vermittelt. Ein cyclisches RGD-Peptid, das an das $\alpha_v\beta_3$-Integrin bindet, wird von der Firma Bachem Biochemica GmbH, Heidelberg, Deutschland, vertrieben.

Eine weitere mögliche Interaktion von TSP-1 mit einem Rezeptor auf Fibroblasten ist die Bindung an das IAP über die C-terminale Zellbindungsdomäne (CBD). Die C-terminale Zellbindungsdomäne (CBD) ist eine Domäne des TSP-1, welche spezifisch mit dem IAP wechselwirkt. Ein verkürztes TSP-1, das nur aus dieser C-terminalen Zellbindungsdomäne besteht und an IAP bindet, wird von der Firma Bachem Biochemica GmbH, Heidelberg, Deutschland, als CBD-Peptid vertrieben.

Die Zugabe des CBD-Peptids und des cyclischen RGD-Peptids führte überraschenderweise zu einer deutlichen Erhöhung der Apoptoserate, die ähnlich hoch war wie die Erhöhung der Apoptoserate durch die Zugabe von TSP-1 (Beispiel 7). Daraus folgt, daß nur eine kombinierte Gabe beider Peptide an IAP und das Integrin $\alpha_v\beta_3$ apoptotisch wirksam ist.

Damit konnte überraschenderweise gezeigt werden, daß die Aktivität des TSP-1 zur Induktion der spontanen Apoptose ausschließlich über die Bindung an IAP und $\alpha_v\beta_3$ vermittelt wird (Beispiel 10).

Da die Fibroblasten in der in-vivo Situation normalerweise nicht Flüssigkeitsströmungen ausgesetzt sind, wurde von den vorstehend benannten Erfindern weiterhin untersucht, ob auch andere mechanische Kräfte die selbstinduzierte Apoptose der Fibroblasten inhibieren können.

Entsprechende Untersuchungen wurden an Fibroblasten durchgeführt, die auf Kollagen-Gelen gezüchtet wurden, die mit Ascorbinsäure isometrisch kontrehiert wurden (Beispiel 11). Dabei wurde überraschenderweise gefunden, daß eine Kultivierung unter Spannung die spontane Apoptose hemmt. Weiterhin wurde überraschenderweise gefunden, daß eine Entspannung solcher unter Anlegen von mechanischer Spannung kultivierten Fibroblasten zu einer sehr deutlichen Erhöhung der Apoptoserste der Fibroblasten führt (Beispiel 12). Die Ergebnisse, die unter Zugabe von Thrombosporidin-1, anti-TSP1 und anti-Rezeptorantikörpern erzielt wurden, stützen diese Befunde und zeigen deutlich, daß überraschenderweise das Anlegen mechanischer Spannung genau wie die dynamische Kultivierung die Apoptoss hemmt. Die entsprechenden Ergebnisse sind In Beispiel 13 gezeigt.

Durch die vorstehend beschriebenen Untersuchungen konnte von den vorstehend genannten Erfindern hier erstmals gezeigt werden, daß allein mechanische Kräfte für die Regulation der Apoptose hinreichend sind. Die zugrunde liegenden molekularen Mechanismen wurden von den vorstehend genannten Erfindern erstmals aufgedeckt.

Überraschenderweise und gänzlich unvorhersehbarer Weise können daher erfindungsgemäß verwendbare Substanzen eingesetzt werden, um einen besonders guten Heilungserfolg bei Wunden zu gewährleisten.

Außerdem können erfindungsgemäß verwendbare Substanzen und mechanische Kräfte kombiniert eingesetzt werden, um einen besonders guten Heilungserfolg bei Wunden zu gewährleisten.

Die vorgestellten Ergebnisse zeigen nämlich, daß die eigentliche Ursache der Apoptoseinduktion das Ausbleiben von auf die Zellen einwirkenden mechanischen Kräften ist, wobei es unerheblich ist, welcher Art diese Kräfte sind. Dadurch werden die Fibroblasten zur Sekretion von TSP-1 veranlaßt, was anschließend durch die Wechselwirkung mit dem Integrin $\alpha_v\beta_3$ und dem IAP zur Induktion der Apoptose führt. Die Abnahme der Fibroblastenpopulation (und anderer Zellen) führt zu dem verlangsamten bzw. ausbleibenden Heilungsverlauf.

Für die Zwecke der vorliegenden Erfindung wird unter Apoptoserate der Anteil der apoptotischen Zellen an der Gesamtzellzahl verstanden. Eine erfindungsgemäß verwendbare Substanz gilt als anti-apoptotisch wirksam, wenn durch ihre Verwendung in der Zellkultur die Apoptoserate verglichen mit einer unter identischen Bedingungen, aber ohne Zusatz eben dieser Substanz durchgeführten Kultivierung um 10%, bevorzugt um mindestens 50%, besonders bevorzugt um mindestens 75% und ganz besonders bevorzugt um mindestens 90% gesenkt werden kann.

Solche Substanzen kann man identifizieren, indem man:

(i) Zellen züchtet, die sowohl IAP als auch das Integrin $\alpha_v\beta_3$ exprimieren,

(ii) die Zellen zur Bildung einer Apoptose-induzierenden Substanz veranlaßt, und/oder eine Substanz bzw. Substanzen zusetzt, die Apoptose induziert/induzieren,

(iii) die Testsubstanz zusetzt,

(iv) die Apoptoserate misst, und

(v) solche Substanzen auswählt, deren Zusatz zur Zellkultur eine verringerte Apoptoserate ergibt,

Zur Durchführung der entsprechenden Experimente werden die Zellen in geeigneten Zellkulturgefäßen in den geeigneten Zellkulturmedien kultiviert. Üblicherweise sind dies Standardmedien, die im Stand der Technik allgemein bekannt sind. Beispielsweise werden die Zellen in DMEM, M-199, IF-Basalmedien usw. kultiviert. Für praktisch alle Zellen und Zellinien sind heute geeignete Kulturmedien vorhanden. Dem Kulturmedium können vor Beginn der Kultur Wachstumsfaktoren und Hormone zugesetzt werden, wie beispielsweise fötales Kälberserum (FCS, fetal calf serum). Die Kultivierung von Säugerzellen erfolgt meist bei 37°C in einer 5%igen $CO_2$-Atmosphäre, die im Zusammenhang mit den in den Zellkulturmedien verwendeten Puffern z.B. Natriumcarbonatpuffern, eine pH-Wert-Stabilisierung des Zellkulturmediums ermöglicht. Weiterhin können den Zellkulturmedien vor Beginn der Kultivierung Antibiotika zugesetzt werden, die spezifisch mit prokaryotischen kontaminierenden Mikroorganismen wechselwirken und deren Wachstum inhibieren, das Wachstum der eukaryotischen Zellen jedoch weitgehend unbeeinflußt lassen und so die Zellkultur vor Kontamination schützen. Weitere Hinweise und Angaben zur Zellzüchtung können Standardwerken entnommen werden, z.B. Zell- und Gewebekultur, 3. Auflage, Toni Lindl, Jörg Bauer, (1994), Gustav Fischer Verlag.

Geeignete Kulturbedingungen für die Kultivierung von Fibroblasten sind in Beispiel 1 angegeben.

Zur Induktion bzw. Erhöhung der Apoptoserate können der Zellkultur Apoptose-Mediatoren zugegeben werden.

Die spontane Apoptoserate in einer statischen Zellkultur ist nämlich relativ gering, verglichen mit Apoptoseraten, die von Mediatoren, wie TNF-$\alpha$, ausgelöst werden. Bei der spontanen Apoptoserate, ausgelöst durch die statische Zellkulturbedingung, werden Apoptoseraten im Bereich von 0,5% bis 12% der Gesamtzellen erreicht. Bei der durch Mediatoren ausgelösten Apoptose können Apoptoseraten von bis zu 100% erreicht werden.

Diese Mediatoren sind Hormone und andere Substanzen, die apoptotisch wirksam sind. Diese Mediatoren werden in gelöster Form der Zellkultur zugegeben, wobei zu beachten ist, daß die verwendeten Lösungen steril sein müssen. Die Sterilität der Lösungen kann auf verschiedene Weisen erreicht werden, vorzugsweise durch Hitzebehandlung (Autoklavieren bei 2 bar und 120°C), oder, wenn dieses Verfahren aufgrund einer besonderen Hitzeempfindlichkeit des Mediators nicht geeignet ist, durch Sterilfiltrieren, beispielsweise mit Nalgene Einmal-Sterilfiltern. Solche Verfahren zur Sterilisierung von Zusätzen für die Zellkultur sind dem Fachmann gut bekannt.

Bei den zugesetzten Testsubstanzen handelt es sich vorzugsweise um monoklonale Antikörper, Antikörperfragmente, polyklonale Antikörper und Peptide. Weiterhin bevorzugte Substanzen, die als Testsubstanzen in dem erfindungsgemäßen Testsystem untersucht werden können, sind niedermolekulare Verbindungen. Solche Verbindungen haben oft keine oder nur geringe Nebenwirkungen, wenn sie als aktives Prinzip in einer pharmazeutischen Zusammensetzung eingesetzt werden. Ein weiterer Vorteil solcher Substanzen ist die Möglichkeit der oralen Verabreichung. Es können jedoch auch andere Substanzen, von denen vermutet wird, daß sie anti-apoptotische Wirkung entfalten können, zugesetzt werden. Diese Substanzen werden vorzugsweise in gelöster Form verabreicht. Die Lösungsmittel müssen hierbei mit der Zellkultur kompatibel sein. Vorzugsweise werden diese Testsubstanzen daher in Pufferlösungen, wie sie allgemein in der Zellkultur weite Verbreitung erlangt haben, gelöst. Beispiele hierfür sind Phosphatpuffer, Natriumcarbonatpuffer und andere. Die gelösten Testsubstanzen werden vorzugsweise vor dem Zusatz in die Zellkultur durch Sterilfiltration (Nalgene Einmal-Sterilfilter) sterilfiltriert (sterilisiert), um kontaminierende Mikroorganismen oder Sporen von Pilzen und ungelöste Bestandteile zu entfernen.

Vorzugsweise wird die gelöste Testsubstanz vor Zusatz zu der Zellkultur temperiert, d.h. der Temperatur der Zellkultur angepaßt. Die Volumina richten sich nach der Konzentration der eingesetzten Testsubstanz, die ereicht werden soll, und vorzugsweise handelt es sich um geringe Volumina, damit keine Verdünnungseffekte in den Zellkulturmedien auftreten. Die Verfahren, mit denen solche Testsubstanzen in Zellkulturen, vorzugsweise in gelöstem Zustand, eingebracht werden können, sind dem Fachmann gut bekannt.

Die Abnahme der Apoptoserate kann durch jedes geeignete Meßverfahren bestimmt werden. Bevorzugt einsetzbar für die vorliegenden Zwecke sind die in der DE 199 52 960.4 beschriebenen Methoden. Besonders geeignet als Frühindikator ist die Messung des Ausbleibens des Calciumeinstroms in die Zelle durch Verwendung von intrazellulären Calciumindikatoren (siehe Beispiel 4). Ein Verfahren, welches geeignet ist, die genauen Apoptoseraten zu bestimmen, ist die Färbung der DNS von apoptotischen Zellen und nachfolgende morphometrische Zellkemanalyse bzw. Analyse des zellulären DNS-Gehalts in einem Durchflußzytometer. Ein Beispiel für einen geeigneten Fluoreszenzfarbstoff stellt DAPI dar. Diese Verfahren sind im Stand der Technik gut bekannt und werden allgemein als Nachweis für apoptotische Zellen verwendet. Nimmt die Anzahl der apoptotischen Zellen nach Einsatz eines potentiellen Inhibitors der Apoptose um mehr als 10%, bevorzugt mehr als 50%, besonders bevorzugt um mehr als 75%, und ganz besonders bevorzugt um mehr als 90% ab, bezogen auf die Anzahl apoptotischer Zellen im Testsystem in statischer Kultur, ggf. nach Zusatz einer Apoptose-induzierenden Substanz, so gilt diese Substanz erfindungsgemäß als Inhibitor der Apoptose bei den verwendeten Zellen.

Als Frühindikator für Screeningzwecke kann das Ausbleiben des Calciumeinstroms in die Zelle verwendet werden. Substanzen, die zum Ausbleiben des Calciumeinstroms führen, müssen anschließend durch DAPI-Färbung hinsichtlich ihrer Wirkung auf die Apoptose-Inhibition überprüft werden (siehe Beispiel 4).

Ferner können auch andere DNS-Farbstoffe (z.B. Hoechst 33258) oder gängige Verfahren, wie der TUNEL-Assay (Tdt-mediated XdUTP nick end labeling; DNS-Brüche), der Nachweis von apoptotischen Enzymen (z.B. PARP, Caspasen) oder Proteinen (z.B. p53, CD95), die Translokation von Phosphatidylserin mit fluoreszierendem Annexin oder der Nachweis der DNS-Leiter im Agarose-Gel eingesetzt werden.

Mit dem offenbarten Verfahren können anti-apoptotisch wirksame Substanzen aufgefunden werden. Bevorzugt sind dies Verbindungen, die entweder an Rezeptoren auf der Zelloberfläche, besonders bevorzugt IAP und/oder das Integrin $\alpha_v\beta_3$, oder an humorale Faktoren, besonders bevorzugt TSP-1, so binden, daß die hier beschriebene spezifische Wechselwirkung zwischen TSP-1 und IAP und $\alpha_v\beta_3$, die zur Induktion der Apoptose führt, nicht stattfindet, so daß die Apoptose nicht induziert werden kann und dadurch ausbleibt.

In einer weiteren bevorzugten Ausführungsform binden diese Substanzen an das IAP und verhindern dadurch den Apoptose-spezifischen Calciumeinstrom in die Zelle, so daß die Apoptose nicht induziert werden kann und somit ausbleibt.

Diese Inhibitoren sind zur Herstellung pharmazeutischer Zubereitungen geeignet, die bei der Wundbehandlung zur Unterdrückung der Apoptose von Fibroblasten, Epithelzellen und anderen Zellen (z.B. glatte Muskelzellen) verwendet werden können.

Aktive mit dem erfindungsgemäßen Verfahren zu testende Wirksubstanzen sind vor allem monoklonale Antikörper und Peptide, die mit gängigen Methoden der Molekularbiologie und der Gentechnologie leicht erhältlich sind. Es ist jedoch klar, daß auch andere Substanzen, die eine entsprechende Wirkung aufweisen, mit dem erfindungsgemäßen Testsystem aufgefunden werden können. Der Begriff "Antikörper" wird hier verwendet zur Beschreibung sowohl von kompletten Antikörpern (d.h. Antikörper, die zwei schwere und zwei leichte Ketten besitzen) als auch von Fragmenten von Antikörpern, die mindestens eine Antigenbindestelle besitzen. Die Identifizierung eines bei Fibroblasten anti-apoptotisch wirksamen Anti-TSP-1-Antikörpers ist in Beispiel 6 beschrieben. In ähnlicher Weise können viele andere Antikörper oder Antikörperfragmente getestet werden, um die Induktion der Apoptose zu verhindern.

Es ist klar, daß eine ganze Reihe von Peptiden und Antikörpern (Antikörperfragmente) hergestellt werden können, die in ähnlicher Weise mit dem erfindungsgemäßen Verfahren als anti-apoptotisch wirksam identifiziert werden können. Darüber hinaus können auch andere Substanzen, die nicht Antikörper (Antikörperfragmente) oder Peptide sind, mit Hilfe des erfindungsgemäßen Verfahrens als anti-apoptotisch wirksam identifiziert werden.

Für die Zwecke der vorliegenden Erfindung wird unter dem Begriff "Peptid" eine Substanz verstanden, die aus einer oder mehrere Ketten von mehreren, d.h. 2 oder mehr, durch Peptidbindungen verbundenen Aminosäuren, besteht.

Für die Zwecke der vorliegenden Erfindung wird unter dem Begriff "Protein" eine Substanz verstanden, in der mehrere "Peptide" durch Peptidbindungen miteinander verbunden sind. Diese Definition umfasst gleichermaßen native Proteine als auch zumindest teilweise "künstliche" Proteine, wobei solche "künstlichen" Proteine z.B. durch Anfügen von chemischen Resten an die Aminosäurekette verändert sein können, die bei nativen Proteinen normalerweise nicht vorkommen.

Für die Zwecke der vorliegenden Erfindung wird unter einer Peptidbibliothek eine Sammlung unterschiedlicher Peptide verstanden, die ohne weiteren technischen Aufwand vom Fachmann auf bestimmte, spezifische Bindungseigenschaften hin untersucht werden kann. Insbesondere gut dem Fachmann bekannt und technisch äußerst einfach zu handhaben sind beispielsweise die auf der sogenannten Phagen-Display-Technologie basierenden Peptidbibliotheken, bei den in wenigen Millilitern Testflüssigkeit bis zu $10^7$ und mehr Phagenpartikel enthalten sein können, die jeweils spezisch ein bestimmtes Peptid auf ihrer Obefläche exprimieren. Werden solche Phagenpafikel einer affinitätschromatographischen Anreicherung unterzogen, so wird mit den spezifisch bindenden Peptiden auch gleichzeitig eine biologische Fabrik für die Produktion, nämlich ein monklonaler Phagenpartikel, d.h. ein Expressionssystem, mitgeliefert. Solche Systeme sind dem Fachmann extrem gut bekannt.

Native Peptide weisen allerdings oft eine geringe metabolische Stabilität gegenüber Peptidasen und eine relativ schlechte Bioverfügbarkeit auf.

Ausgehend von den oben gezeigten Peptiden kann der Fachmann ohne erfinderisches Zutun eine ganze Reihe von abgeleiteten Verbindungen entwickeln, die eine ähnliche oder gleichartige Wirkungsweise besitzen, und die u.a. auch Peptidmimetica genannt werden.

Als Peptidmimetica werden dabei für die Zwecke der vorliegenden Erfindung Verbindungen bezeichnet, welche die Struktur von Peptiden nachahmen und als Liganden in der Lage sind, die biologische Aktivität auf Rezeptor-/Enzymebene zu imitieren (Agonist) oder zu blockieren (Antagonist). Vor allem sollen die Peptidmimetica eine verbesserte Bioverfügbarkeit und eine verbessert metabolische Stabilität aufweisen.

Im Prinzip bieten sich folgende Möglichkeiten zur Mimetisierung/Derivatisierung einer Peptidstruktur an:

- Verwendung von D - statt L -Aminosäuren

- Modifizierung der Seitenkette von Aminosäuren

- Veränderung/Verlängerung der Peptidhauptkette

- Cyclisierung zur Konformationsstabilisierung

- Verwendung von Templaten, die eine bestimmte Sekundärstruktur erzwingen

Während die proteolytische Stabilität eines Peptids durch Austausch von L - gegen D -Aminosäuren gesteigert werden kann, führt die Modifikation der Seitenketten einer der Aminosäuren oft zu einer Verbesserung der Bindungseigenschaften des gesamten Peptids.

Bei der Veränderung des Peptidrückgrates erfolgt in der Regel ein Austausch einer Amidgruppe gegen amidähnliche Gruppierungen ( J. Gante, Angew. Chem. 106 (1994),1780-1802). Durch diese Maßnahmen lassen sich sowohl die Bindungsaffinität als auch die metabolische Stabilität des nativen Peptids beeinflussen.

Durch die Cyclisierung eines linearen Peptids wird dessen Flexibilität und damit dessen globale Konformation festgelegt. Bei Fixierung der biologisch aktiven Konformation wird die Affinität des Peptids zum Rezeptor erhöht, da die Entropieabnahme bei der Bindung kleiner ist als bei der Bindung eines flexiblen, linearen Peptids. Zu diesem Zweck werden Aminosäureseitenketten, die nicht an der Rezeptorerkennung beteiligt sind, miteinander oder mit dem Peptidgerüst verknüpft.

Die Sekundärstruktur des Peptids spielt eine entscheidende Rolle für die molekulare Erkennung des Rezeptors. Neben $\alpha$-Helix und $\beta$-Faltblatt sind sogenannte Turns als Wendepunkte in der Peptidkette wichtige Konformationselemente. Der Ersatz dieser Struktureinheiten durch einen Baustein, welcher nach dem Einfügen in ein Peptid eine definierte Sekundärstruktur stabilisiert, hat zum Konzept des Sekundärstrukturmimeticum geführt.

Auch die Wasserlöslichkeit der Peptide kann beispielsweise durch Einführung von S- und C- Glycopeptid Derivaten erhöht werden. Weitere Maßnahmen können beispielsweise die PEGylierung der Peptide sein.

Auch die Lipophilie der Hexapeptide kann erhöht werden, indem beispielsweise Phenylalanine an die Peptidsequenz angehängt werden.

Die Cyclisierung bzw. die N-terminale Modifikation von Peptiden wird z.B. von Borchard, Journal of controlled Release 62 (1999), 231 -238 und von Blackwell et al., J. Org. Chem. 10 (2001), 5291-302 beschrieben.

Es ist daher klar, daß der Fachmann ausgehend von dem durch die vorliegende Erfindung vermittelte Wissen mit Leichtigkeit zu einer ganzen Reihe von abgeleiteten Peptidmimetika gelangen kann, die jedoch alle von der vorliegenden Erfindung mitumfasst sind.

Unter einem weiteren bevorzugten Gesichtspunkt stellt die vorliegende Erfindung daher auch Peptidmimetica zur Verfügung, die aus den SEQ ID Nos 1-11 abgeleitetet sind, sowie Substanzen, die solche Peptidmimetica umfassen.

Besonders bevorzugte erfindungsgemäß verwendbare Substanzen sind niedermolekulare Verbindungen. Solche Verbindungen haben oft keine oder nur geringe Nebenwirkungen, wenn sie als aktives Prinzip in einer pharmazeutischen Zusammensetzung eingesetzt werden. Ein weiterer Vorteil solcher Substanzen ist die Möglichkeit der oralen Verabreichung.

Beispiele hierfür sind cyclische Pentapeptide, wie sie von Haubner et. al., J. Am. Chem. Soc. 1996, 118, 7641-7472, beschrieben wurden. Erfindungsgemäß werden den niedermolekularen Stoffen kleine Peptide, Aminosäuren und Aminosäureanaloga, Steroide, Nukleotide und weitere organisch-chemische Stoffe mit einem Molekulargewicht von $\leq$ 5000 bevorzugt $\leq$ 3000 und besonders bevorzugt $\leq$ 2000 zugerechnet. [Haubner, R., Gratias, R., Diefenbach, B., Goodman, S. L., Jonczyk, A., Kessier, *II., Structural and Functional Aspects of RGD-Containing Cyclic Pentapeptides as Highly Potent and Selective Integrin $\alpha_v\beta_3$ Antagonists,* 118, 746 1-7472 (1996)].

Kombinatorische Bibliotheken sind dem Fachmann ebenfalls gut bekannt. Es existieren eine Vielzahl solcher Bibliotheken, die eine sehr große Anzahl sehr unterschiedlicher Moleküle beinhalten. Aus diesen Bibliotheken können in automatisierten Verfahren sehr spezifisch bindende, niedermolekulare Verbindungen gewonnen werden, und es ist klar, daß es angesichts der hier vorgestellten Erfindung mit Leichtigkeit möglich ist, entsprechende, erfindungsgemäß verwendbare Identifikate zu erzeugen.

Die Herstellung entsprechender Präparate erfolgt nach üblichen Verfahren. Beispielsweise können Peptide oder Antikörper (Antikörperfragmente), die aktiver Bestandteil einer pharmazeutischen Zubereitung sind, in einem pharmazeutisch annehmbaren Träger gelöst werden. Ein Beispiel für einen pharmazeutisch annehmbaren Träger können Pufferlösungen wie Phosphatpuffer oder Citratpuffer, sein. Zur Aufrechterhaltung der Aktivität der Peptide können auch Reagentien zugesetzt werden, die pharmazeutisch annehmbar sind und beispielsweise ein reduzierendes Milieu in der pharmazeutischen Zubereitung aufrechterhalten.

Die spezifische Dosierung und Posologie ist für jeden Patienten von einer Anzahl von Faktoren abhängig, einschließlich der Aktivität der verwendeten spezifischen Verbindungen, dem Alter des Patienten, dem Körpergewicht, des allgemeinen Gesundheitszustands, des Geschlechts, der Ernährung, der Zeit der Verabreichung, dem Weg der Verabreichung, der Exkretionsrate, der Verbindung mit anderen Arzneimitteln und der Schwere der einzelnen Erkrankung, für die die Therapie angewandt wird. Sie wird von einem Arzt in Abhängigkeit von diesen Faktoren ermittelt.

Polypeptidmedikamente, z.B. Proteinmedikamente oder Antikörpermedikamente, werden üblicherweise parenteral verabreicht, z.B. durch ein Inhalationsspray, rektal, durch subkutane, intravenöse, intramuskuläre, intraartikuläre und intrathecale Injektions- und Infusionstechniken, oder äußerlich in pharmazeutischen Formulierungen, welche konventionelle, pharmazeutisch annehmbare Träger, Adjuvantien und Vehikel enthalten. Je nach Art der identifizierten Substanz kommen auch andere Verabreichungswege in Betracht, z.B. oral.

Die Erfindung stellt ebenso pharmazeutische Zusammensetzungen bereit, die eine wirksame Menge einer anti-apoptotisch wirksamen Substanz in Kombination mit einem konventionellen pharmazeutischen Träger umfassen. Ein pharmazeutischer Träger ist z.B. ein fester oder flüssiger Füllstoff, ein Verkapselungsmaterial oder ein Lösungsmittel. Beispiele für Materialien, die als pharmazeutische Träger dienen können, sind Zucker wie Lactose, Glucose und Saccharose; Stärke wie Malsstärke und Kartoffelstärke; Cellulose und deren Derivate wie Natriumcarboxymethylcellulose, Ethylcellulose und Celluloseacetat; pulverisierter Tragacanth; Malz; Gelatine; Talg; Arzneimittelträger wie Kakaobutter und Zäpfchenwachse; Öle wie Erdnußöl, Baumwollsamenöl, Färberdistelöl. Sesamöl, Olivenöl, Maisöl und Sojabohnenöl; Polyole wie Propylenglykol, Glycerin, Sorbitol, Mannitol und Polyethylenglykol; Ester wie Ethyloleat und Ethyllaureat; Agar; Puffermittel wie Magnesiumhydroxid und Aluminiumhydroxid; Alginsäure; Pyrdgen-freies Wasser, isotonische Salzlösung; Ringers Lösung, Ethylalkohol und Phosphatpufferl6sungen wie auch andere nichtioxische kompatible Substanzen, die in pharmazeutischen Formulierungen verwendet werden. Benetzungsmittel, Emulgatoren und Gleitmittel wie Natriumlaurylsulfat und Magnesiumstearat, wie auch Färbemittel, Gleitmittel, Deschichtungsmittel sowie Parfümierungsmittel und Konservierungsstoffe können ebenso in den Zubereitungen vorhanden sein, entsprechend den Anforderungen des Galenikers. Die Menge des aktiven Wirkstoffes, der mit den Trägermaterialien kombiniert wird, um eine Einzeldosis zu produzieren, wird abhängig von dem behandelten Patienten und der speziellen Methode der Verabreichung variieren. Ein Beispiel für eine solche pharmazeutische Formulierung ist in Beispiel 19 gezeigt.

Die Herstellung einer sogenannten lebenden Haut ist beispielsweise in den Anmeldungen EP 1 005 873 und WO 99/43787 der Advanced Tissue Sciences Inc. genauestens beschrieben, und sie ist dem Fachmann geläufig. Für die Zwecke der vorliegenden Erfindung kann jede wie auch immer geartete künstliche Haut verwendet werden, wenn auf ihr autologe oder heterologe Hautzellen kultiviert werden, die auf ihrer Oberfläche CD47 oder $\alpha_v\beta_3$ exprimieren, und wenn die die Haut tragende Matrix verstreckbar ist.

Die nachstehenden Beispiele erläutern die Erfindung näher. Sie sollen jedoch nicht einschränkend verstanden werden.

**Abkürzungen**

IAP:        Integrin-assoziiertes Protein (CD 47)

CBD:        C-terminale Zellbindungsdomäne des TSP-1

TSP-1:      Thrombospondin 1

RGD:        Arg-Gly-Asp

HUVEC:      Humane Nabelschnur-Venen-Endothelzellen

HPEC:       Humane Plazentaendothelzellen

DAPI:       4',6'-Diamidino-2-phenylindol

MRC-5       Fibroblastenzelllinie

Milli-Q     gereinigtes Wasser

TNF-$\alpha$      Tumomekrosefaktor alpha

bFGF        basischer Fibroblastenwachstumsfaktor

Bevorzugt handelt es sich bei den erfindungsgemäß verwendbaren Substanzen um spezifische Antikörper oder funktionelle Varianten hiervon gegen Thrombospondin 1, IAP und/oder Integrin $a_v\beta_3$ ist.

Weiterhin bevorzugt handelt es sich bei den erfindungsgemäß verwendbaren Substanzen um Peptide.

Besonders bevorzugt handelt es sich bei den erfindungsgemäß verwendbaren Substanzen um Peptide ausgewählt ist aus den in den SEQ ID NOs 1 bis 11 dargestellten Aminosäuresequenzen bzw. davon abgeleiteten Peptidmimetika oder diese Sequenzen umfassende Proteine.

Unter einem weiteren besonders bevorzugten Gesichtsopunkt der vorliegenden Erfindung könnenerfindungsgemäß verwendbare Peptide aus einer Peptidbibliothek durch Vorscreenen mit Hilfe affinitätschromatographischer Verfahren unter Verwendung von Thrombospondin 1, IAP und/oder Integrin $\alpha_v\beta_3$ als Target ausgewählt werden.

Weiterhin bevorzugt handelt es sich bei den erfindungsgemäß verwendbaren Substanzen um niedermolekulare Wirkstoffe mit einem Molekulargewicht von $\leq 5000$ ist.

Bevorzugt wird der niedermolekulare Wirkstoff aus einer kombinatorischen Bibliothek durch Vorscreenen mit Hilfe affinitätschromatographischer Verfahren unter Verwendung von Thrombospondin 1, IAP und/oder Integrin $a_v\beta_3$ als Target ausgewählt.

Die in dem Identifikationsverfahren eingesetzten Zellen können Endothelzellen, bevorzugt humane Nabelschnur-Venen-Endothelzellen (HUVEC), humane plazentale Endothelzellen (HPEC), bovine Aortenendothelzellen (BABC), Schweinehirnkapillarendothelzellen (PBMEC), Hybridzellinien (EA.hy 926) und humane mikrovaskuläre Endothelzellen (HMVEC) sowie glatte Muskelzellen oder Fibroblasten sein.

Die in dem Identifikationsverfahren eingesetzten Zellen können weiterhin auch gentechnisch veränderte Zellen sein, die sowohl IAP als auch $\alpha_v\beta_3$ auf ihrer Oberfläche exprimieren.

Besonders bevorzugt werden die in demerfindungsgemäßen Identifikationsverfahren eingesetzten Zellen unter Bedingungen kultiviert, unter denen gleichbleibend gerichtete laminare Strömungen nicht auftreten.

Unter einemm weiteren Gesichtspunkt der vorliegenden Erfindung wird zur Durchführung des Identifikationsverfahren der Zellkultur Thrombospondin 1 oder eine analoge Verbindung mit gleichen Bindungseigenschaften zugesetzt.

In dem Identifikationsverfahren kann die Auslösung der Apoptose durch Messung des erhöhten Calciumeinstroms in die Zelle bestimmt wird.

In dem Identifikationsverfahren kann die Apoptoserate weiterhin in an sich bekannter Weise durch DAPI-Färbung, TUNEL-Assay, DNS-Leiter, Annexin-Färbung oder einen Enzymnachweis bestimmt wird.

Die erfindungsgemäßen Identifikate binden erfindungsgemäß bevorzugt an einen Calciumkanal in der Zellmembran so, daß der Apoptose-spezifische Calciumeinstrom in die Zellen unterbunden wird, wobei bevorzugt IAP an der Bildung des Calciumkanals beteiligt ist.

Bevorzugt wird dabei der Apoptose-spezifische Calciumeinstrom in Fibroblasten hemmt

Dem erfindungsgemäß herstellbaren Medikament wird (werden) bevorzugt zusätzlich zu dem Identifikat ein oder mehrere unterschiedliche(r) Fibroblastenwachstumsfaktor(en), bevorzugt bFGF, beigemischt.

Unter einem weiteren Gesichtspunkt der vorliegenden Erfindung betrifft diese ein Verfahren zur Herstellung einer lebenden Ersatzhaut, wobei die Ersatzhaut nach Anwachsen der Zellen auf einer biokompatiblen Membran um 10% bis 90% verstreckt wird.

Unter einem weiteren bevorzugten Gesichtspunkt betrifft die Erfindung ein Verfahren zur Herstellung einer lebenden Ersatzhaut, wobei die Ersatzhaut vor der Applikation der Ersatzhaut auf einer Wunde um 10% bis 90% verstreckt wird.

Unter einem weiteren bevorzugten Gesichtspunkt betrifft die Erfindung ein Verfahren zur Herstellung einer lebenden Ersatzhaut, wobei die Ersatzhaut nach Anwachsen der Zellen auf einer biokompatiblen Membran um 10% bis 90% verstreckt wird und wobei ein oder mehrere niedermolekulare Identifikat(e) in Kontakt mit dieser Ersatzhaut gebracht wird (werden).

Unter einem weiteren bevorzugten Gesichtspunkt betrifft die Erfindung ein Verfahren zur Herstellung einer lebenden Ersatzhaut, wobei die Ersatzhaut vor der Applikation der Ersatzhaut auf einer Wunde um 10% bis 90% verstreckt wird und wobei und wobei ein oder mehrere niedermolekulare Identifikat(e)in Kontakt mit dieser Ersatzhaut gebracht wird (werden).

Zusätzlich kann die lebende Haut bevorzugt mit einem Fibroblastenwachstumsfaktor, bevorzugt bFGF, in Kontakt gebracht werden.

**Kurze Beschreibung der Figuren:**

Fig. 1:     Streckapparatur, Ansicht von oben

Fig. 2:     Streckapparatur, Ansicht von der Seite

Fig. 3:     schematische Funktionsweise der Streckapparatur (Bsp. 16)

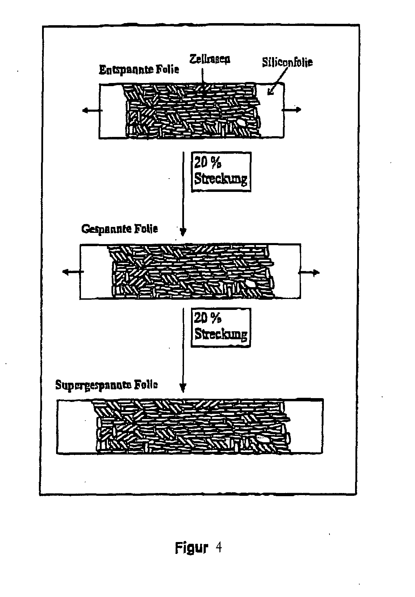

Fig. 4:     schematische Darstellung der zweifachen Dehnung von Zellen (Bsp, 17)

Fig. 5:     Bestimmung des Einflusses von zweifacher Dehnung der Zellen auf den Apoptoseindex (Bsp. 17)

Fig. 6.     Graphische Darstellung des Verlaufes des Apoptose- und Proliferationsindexes nach 20 % Spannen In Abhängigkeit von der Zeit. (Bsp. 18)

BEISPIEL 1

Kultivierung von MRC-5 Fibroblasten (ATCC-Nr.: CCL-171):

A) Verwendete Reagenzien und Antikörper:

Die RGD Peptide, das CBD Peptid und das Tsp1 werden entsprechend den Angaben in der folgenden Tabelle verwendet:

| Ligand | Konzentration | Inkubationszeit | Biologische Aktivität |
|---|---|---|---|
| humanes Thrombospondin 1 Prof. Dr. Vischer, Universität Münster, Institut für Artherioskleroseforschung, | 1 μg/ml | (2-)24-72 h | bindet Integrin $\alpha_v\beta_3$ und IAP |
| RGD-cyclisch Bachem Biochemica GmbH, Heidelberg, Deutschland Katalog-Nr H25740 | 250 μg/ml | (2-)24-72 h | bindet Integrin $\alpha_v\beta_3$ |
| IAP-Peptid (CBD) Bachem Biochemica GmbH, Heidelberg, Deutschland Katalog-Nr H1418 | 400 μg/ml | (2-)24-72 h | bindet IAP |

Spezifische Antikörper:

1.) monoklonaler Antikörper aus Maus gegen humanes CD47, von Cymbus Biotechnology LTD, UK, Katalog.-Nr.: CBL 489, 50 μg/ml.

2.) monoklonaler und polyklonaler Antikörper gegen Tsp1, von Prof. Dr. Vischer, Universität Münster, Institut für Artherioskleroseforschung, Deutschland, 50 μg/ml.

3.) monoklonaler Antikörper gegen die Integrinuntereinheit $\alpha_V$, Chemicon International Inc., Kanada, Katalog-Nr.: MAB1960, 50 μg/ml.

B) Statische, dynamische und gespannte Kultivierung von MRC-5-Fibroblasten

Kulturmedium für MRC-5:

89 ml IF-Basalmedium

10 ml FCS (Fötales Kälber Serum)

1 ml L-Glutaminstammlösung

IF-Basaimedium

Das IF-Basalmedium ist eine 1:1 Mischung von IMDM (Iscove's Modifiziertes Dulbecco's Medium) und Ham's F12-Medium.

L-Glutamin-Stammfösung

200 mM L-Glutamin werden in IF-Basalmedium gelöst und sterilfiltriert.

B1) statische Kultivierung (ohne Strömungskräfte im Zellkulturmedium):

Die verwendete Zellinie MRC-5 wird in ungelatinierten Zellkulturgefäßen ausgesät. Die anschließende Kultivierung erfolgt im Brutschrank bei 37°C und 5 Vol% $CO_2$ in wasserdampfgesättigter Atmosphäre. Ein Wechseln des Kulturmediums erfolgt jeden zweiten bis dritten Tag, und nach Erreichen der Konfluenz werden die Zellen mit einer Teilungsrate von 1:5 bis 1:10 passagiert.

B2) dynamische Kultivierung (Scherkräfte im Zellkulturmedium):

Die Fibroblasten werden zunächst unter statischen Bedingungen bis zum Erreichen der Konfluenz kultiviert. Anschliessend erfolgt die Kultivierung für 24 h unter dynamischen Bedingungen in der Plattenkegelscherungsapparatur (nach Bussolori *et al*. (1982) Rev. Sci. Instrum. 53, 1851-1854).

Dazu werden die Zellen zunächst in gelatinierten Kulturschalen (Ø = 35 mm) mit 0,15 ml/cm$^2$ Kulturmedium vorkultiviert und danach für maximal 24 h unter turbulenten bzw. laminaren Strömungsbedingungen in der Plattenkegelscherungsapparatur weiterkultiviert. Die Strömungsbedingungen resultieren aus dem Winkel des Kegels. Bei einem Kegelwinkel von 5° werden die Zellen turbulent überströmt, bei einem Kegelwinkel von 0,5° laminar.

Lösungen:

70 % (v/v) Ethanol

Kulturmedium für MRC-5-Fibroblasten (s.o.)

Material:

Plattenkegelscherungsapparatur (nach Bussolori *et al*. (1982) Rev. Sci. Instrum. 53, 1851-1854)

Der erzeugte Scherstress errechnet sich nach: $\tau = \dfrac{\mu \cdot 2\pi \cdot U_{Anzeige}}{\alpha}$

$\tau$ = Scherstress [dyn/cm]

$\mu$ = Viskosität des Mediums bei 37°C

$\alpha$ = Kegelwinkel

$U_{Anzeige}$ = angezeigte Drehzahl am

Steuerelement [U/min]

Durchführung:

Vor ihrem Einsatz wird die Plattenkegelscherungsapparatur mit einem weichen Tuch und 70 % (v/v) Ethanol gereinigt, der Kegel sterilisiert und die Apparatur auf 37°C im Wärmeschrank temperiert. Die vorkultivierten Zellen werden mit Basalmedium gewaschen, mit 0,1 ml/cm$^2$ frischem Kulturmedium versehen und die Kulturschale zügig in die Apparatur eingebaut.

Der Kegel wird mit Hilfe des Grobtriebes hochgestellt und die Kulturschale samt Deckel in die dafür vorgesehene Fassung eingesetzt. Der Deckel wird abgenommen und der Kegel über der Kulturschale justiert. Der exakte Abstand der Plattenkegelspitze zum Zellrasen wird mit einer Micrometerschraube eingestellt.

Die Skala der Micrometerschraube zeigt dabei einen Wert von 175 an. Bei diesem Wert, der zuvor empirisch ermittelt wurde, dreht sich die Kegelspitze in minimalem Abstand zum Zellrasen, ohne auf diesem zu schleifen. Die zusammengebaute Plattenkegelscherungsapparatur wird zur laminaren bzw. turbulenten Kultivierung der Zellen in den Inkubator bei 37°C, 5 % (v/v) $CO_2$ und wasserdampfgesättigter Atmosphäre gestellt. MRC-5 konditioniertes Medium wird wie in Beispiel 2 gezeigt hergestellt.

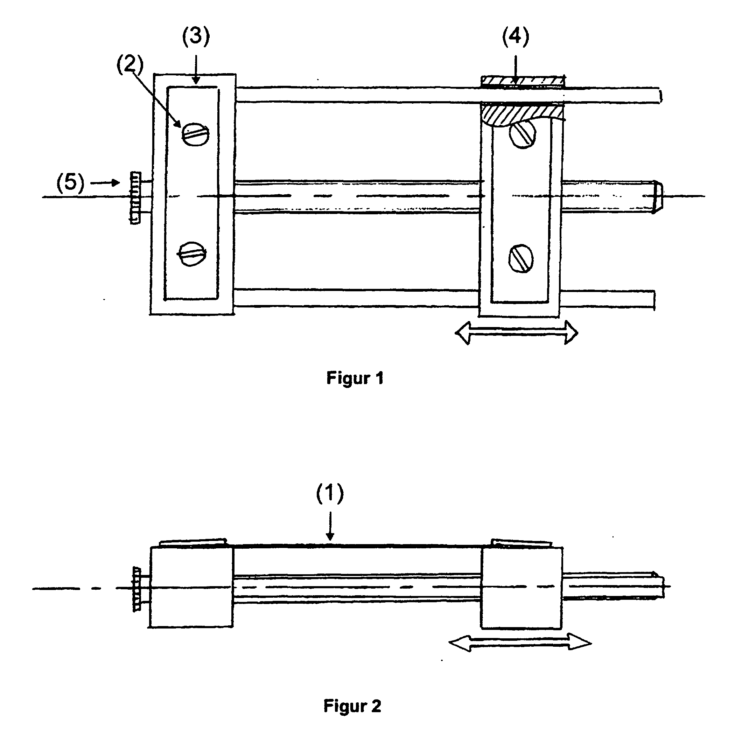

B3) Kultivierung in der Streckapparatur (Eigenbau, Abb. 1 und 2):

Die Zellen werden zunächst auf gelatinierten Silikonfolien (1) vorkultiviert und zwei Tage nach Erreichen der Konfluenz über den gewünschten Zeitraum in der Streckapparatur weiterkultiviert.

Lösungen:

Kulturmedium für MRC-5-Fibroblasten (s.o.)

Material:

Streckapparatur, Zellkulturschalen (Durchmesser: 147 mm) Innensechskantschlüssel, Lineal mit Millimeterskala, Silikonfolie der Typen LP 500-1, LP 500-3, LP 500-5; hergestellt von Laboratoire Perouse Implant, Bornel (Frankreich) Vertrieb durch Aromando Medizintechnik GmbH, Düsseldorf, Deutschland

Durchführung:

Die Streckapparatur wird zunächst mit einem weichen Tuch und 70 % (v/v) Ethanol gereinigt und durch Autoklavieren bei 121 °C und 2 bar für 20 min in gesättigter Wasserdampfatmosphäre sterilisiert.

Die sterile Streckapparatur wird nun so in eine Zellkulturschale (Ø = 147 mm) gelegt, dass die Schrauben (2), die zur Fixierung der Silikonfolie (1) dienen, nach oben zeigen. Die Silikonfolie wird mittels einer Pinzette in die dafür vorgesehene Halterung (3,4) eingeführt und durch Festschrauben der Halterung (3, 4) in dieser fixiert. Die Apparatur wird 180° um ihre horizontale Achse gedreht.

Die Folie wird durch Drehen der Gewindeschraube (5) um die gewünschte Länge gespannt und die Längenänderung mit Hilfe eines unter die Kulturschale gelegten Lineals verfolgt. Abschließend werden 60 ml Kulturmedium in die Kulturschale gegeben. Die zusammengebaute Streckapparatur wird zur Kultivierung der Zellen in den Inkubator bei 37°C. 5 % (v/v) $CO_2$ und wasserdampfgesättigter Atmosphäre gestellt.

BEISPIEL 2

Herstellung von konditioniertem Medium

Lösungen:

Kulturmedium für MRC-5-Fibroblasten (s.o,)

Materialien :

MRC-5 konfluent

Greinerröhrchen

Durchführung:

IF-Basalmedium aus Beispiel 1 wird auf einen konfluenten Zellrasen von MRC-5 gegeben und für 48 Stunden - 72 Stunden konditioniert. Anschließend wird das konditionierte Medium 5 Minuten bei 1000 $g_{av}$ zentrifugiert. Das konditionierte Medium wird bis zum Einsatz bei -20°C eingefroren. Das konditionierte IF-Basalmedium wird für Apoptoseuntersuchungen eingesetzt. Hierfür kann es erneut mit 2 mM Glutamin angereichert werden.

BEISPIEL 3

Bestimmung der Apoptoserate durch Färbung apoptotischer Zellen mit DAPI

DAPI gehört zur Indolfarbstoffgruppe und ist ein selektiver DNS-Farbstoff. Der Farbstoff wird bei 340-360 nm angeregt, und das Emissionsmaximum liegt bei 480 nm. Er wird für Apoptoseuntersuchungen eingesetzt [vgl. Cohen *et al.*, Immunology Today, 14, No. 3, 126-130, 1993)].

Morphologische Auswertung:

Lösungen:

PBS (Phosphate Buffered Saline):

140 mM NaCl, 3 mM KCl, 8 mM $Na_2HPO_4$ und 1,5 mM $KH_2PO_4$ werden in Wasser gelöst, wobei sich ein pH-Wert von 7,2 - 7,4 einstellt. Die erhaltene Lösung wird durch Autoklavieren sterilisiert.

Formaldehydlösung:

4% (v/v) Formaldahyd in PBS

DAPI-Lösung:

30 nM DAPI (Molecular Probes, Leiden, Holland) in MeOH, bei 4°C gelagert.

Materialien:

Petrischale (35 mm) mit MRC-5-Fibroblasten in Kultur

Durchführung:

Der Kulturüberstand einer Petrischale wird abgesaugt, der Zellrasen wird 15 Minuten mit 1 ml Formaldehydlösung auf Eis fixiert, zweimal mit 2 ml PBS gewaschen, für 15 Minuten mit 0,5 ml DAPI-Lösung versetzt, mit PBS gewaschen und unter dem Fluoreszenzmikroskop ausgewertet. Es wird mit dem UV-Filtersatz und einem 20 x oder 40 x Objektiv gearbeitet. 500-1000 Zellen werden zufällig ausgewählt und die Zellen mit apoptotischen Kernen gezählt.
Der Apoptose-Index wird nach folgender Formel berechnet:

$$\text{Apoptose-Index [\%]} = \frac{\text{Anzahl apoptotischer Zellen}}{\text{Gesamtzellzahl}} * 100$$

BEISPIEL 4

<u>Messung des ausgelösten Calciumeinstroms in die Zellen durch Verwendung des intrazellulären Calciumindikators Fluo-3 AM</u>

Fluo-3 ist ein Calciumindikator, der nach Bindung von $Ca^{2+}$ einen fluoreszierenden Komplex bildet. Der Ester Fluo-3 AM wird durch Diffusion von der Zelle aufgenommen. Erst nach Hydrolyse des Esters in der Zelle entsteht der Calciumindikator Fluo 3. Extrazellulärer Farbstoffester beeinträchtigt die Messung daher nicht. Die Messung erfolgt mit normalen Fluorescein-Filtern. Bei einer Anregungswellenlänge von 488 nm (500 nm) liegt das Emissionsmaximum bei 525 nm und erhöht sich durch Calcium Bindung um einen Faktor 100 (200). Der Meßbereich liegt zwischen 0,05 und 20 µM freiem $Ca^{2+}$ [Merritt, J.E. *et al., Biochem. J.* **269,** 513-519 (1990)].

<u>Lösungen:</u>

PBS (s.o.)
Kulturmedium für MRC-5-Fibroblasten (s.o.)

6 mM Fluo-3 AM Stammlösung (50 µg in 10 µl Dimethylsulfoxid (DMSO); Molecular Probes, Leiden)

Fluo-3 AM Einsatzkonzentration: 3 µM in serumfreiem Medium

<u>Materialien:</u>

Zellen in Kultur

24 Loch-Zellkulturplatten (Mikrotiterptatten)

<u>Durchführung:</u>

Konfluente Zellrasen von MRC-5 in 24 Loch-Platten werden mit serumfreiem Medium dreimal gespült, für 30 Minuten mit vorgewärmtem serumfreiem Medium mit Fluo-3 AM unter Kulturbedingungen inkubiert, dreimal gespült und die Platte mit einem Fluorimeter gemessen. Zunächst wird die Empfindlichkeit des Geräts justiert und anschließend werden alle 1-10 Sekunden Fluoreszenzwerte aufgenommen, die dem momentanen Calciumspiegel in der Zelle entsprechen. Während die Messung abläuft, können Substanzen zu den Zellen gegeben werden. Hat eine Substanz einen Einfluß auf den Calciumspiegel, kann man das an der Erhöhung bzw. Erniedrigung der Fluoreszenzwerte erkennen. Mit diesem Verfahren ist eine Beurteilung des Calciumspiegels möglich. Der Calciumeinstrom ist ein frühes Anzeichen für eine stattfindende Apoptose.

BEISPIEL 5

<u>Einfluß konditionierten Mediums auf die Apoptose statisch und dynamisch kultivierter MRC-5 Fibroblasten:</u>

<u>Lösungen und spezifische Materialien:</u>

siehe Beispiel 1

<u>Durchführung:</u>

Das verbrauchte Kulturmedium einer konfluenten MRC-5-Fibroblasten-Kultur wird entfernt, der Zellrasen einmal mit Kulturmedium gewaschen und unter den angegebenen Bedingungen für 24 h kultiviert. Die erhaltenen Ergebnisse sind in der folgenden Tabelle aufgeführt.

| Kultivierung | Kulturmedium | Apoptose [%] |
|---|---|---|
| statisch | frisch | 2,8 ± 0,2 |
| statisch | konditioniert | 5,1 ± 0,2 |
| dynamisch | frisch | 0,5 ± 0,1 |
| dynamisch | konditioniert | 0,5 ± 0,1 |
| dynamisch | Frisch + TSP-1 | 0,4 ± 0,1 |

BEISPIEL 6

Einfluß von Thrombospondin-1 und anti-Tsp1 Antikörpern im

Kulturmedium:

Lösungen und spezifische Materialien:

    siehe Beispiel 1

Durchführung:

Das verbrauchte Kulturmedium einer konfluenten MRC-5-Fibroblasten-Kultur wird entfernt, der Zellrasen einmal mit Kulturmedium gewaschen und unter den angegebenen Bedingungen für 24 h kultiviert. Die erhaltenen Ergebnisse sind in der folgenden Tabelle aufgeführt.

| Kulturmedium | Apoptose [%] |
|---|---|
| frisch | 1,6 ± 0,2 |
| frisch + Tsp1 | 5,6 ± 0,2 |
| konditioniert | 5,1 ± 0,2 |
| frisch + polyklon. anti-Tsp1 | 0,6 ± 0,2 |
| konditioniert + polyklon. anti-Tsp1 | 0,6 ± 0,2 |
| konditioniert + monoklon. anti-Tsp1 | 0,6 ± 0,2 |

BEISPIEL 7

Änderung der Apoptose bei MRC-5 Fibroblasten nach Zugabe spezifischer Peptide in das Kulturmedium:

Lösungen und spezifische Materialien:

    siehe Beispiel 1

Durchführung:

Das verbrauchte Kulturmedium einer konfluenten MRC-5-Fibroblasten-Kultur wird entfernt, der Zellrasen einmal mit Kulturmedium gewaschen und unter den angegebenen Bedingungen für 24h kultiviert. Die erhaltenen Ergebnisse sind in der folgenden Tabelle aufgeführt.

| Kulturmedium | Apoptose [%] |
|---|---|
| frisch | 1,6 ± 0,2 |
| konditioniert | 5,1 ± 0,2 |
| frisch + Tsp1 | 5,6 ± 0,2 |
| frisch + CBD + RGD/cycl. | 5,7 ± 0,3 |

BEISPIEL 8

Tsp1 Sekretion unter statischen und dynamischen Kulturbedingungen (24h):

Durchführung:

Das verbrauchte Kulturmedium einer 1 bis 3 Tage postkonfluenten MRC-5-Fibroblasten-Kultur wird entfernt, der Zellrasen einmal mit Kulturmedium gewaschen und unter den angegebenen Bedingungen für 24 h kultiviert. Das resultierende Medium wird entfernt, bei $1000g_{av}$ 5 min bei Raumtemperatur zentrifugiert und der Überstand zur Bestimmung des Tsp1 Gehalts verwendet. Die Zellzahl der jeweiligen Kultur wird ermittelt. Die erhaltenen Ergebnisse sind in der folgenden Tabelle aufgeführt.

| Kulturbedingung | Tsp1 [ng / 1x10$^6$ Zellen] |
|---|---|
| statisch | 53,5 ± 6,1 |
| dynamisch | 10,9 ± 0,5 |

BEISPIEL 9

Tsp1 Sekretion unter statischen Bedingungen in Abhängigkeit von der Kultivierungsdauer:

Durchführung:

Das verbrauchte Kulturmedium einer konfluenten Kultur wird entfernt, der Zellrasen einmal mit Kulturmedium gewaschen und unter statischen Bedingungen weiterkultiviert. Das resultierende Medium wird entfernt, bei $1000g_{av}$ 5 min bei Raumtemperatur zentrifugiert und der Überstand zur Bestimmung des Tsp1 Gehalts verwendet. Die Zellzahl der jeweiligen Kultur wird ermittelt. Die erhaltenen Ergebnisse sind in der folgenden Tabelle aufgeführt.

| Tage, postkonfluent | Tsp1 [ng / 1x10$^6$ Zellen] |
|---|---|
| 1 | 7,8 ± 3,0 |
| 2 | 19,1 ± 2,5 |
| 3 | 21,2 ± 3,7 |
| 4 | 39,7 ± 3,8 |
| 5 | 99,2 ± 19,7 |
| 6 | 179,6 ± 16,3 |
| 7 | 192,4 ± 18,0 |

BEISPIEL 10

Einfluß von anti-Rezeptor Antikörpern:

Lösungen und spezifische Materialien:

siehe Beispiel 1

Durchführung:

Das verbrauchte Kulturmedium einer konfluenten Kultur wird entfernt, der Zellrasen einmal mit Kulturmedium gewaschen und unter den angegebenen Bedingungen für 24 h kultiviert. Die erhaltenen Ergebnisse sind in der folgenden Tabelle aufgeführt.

| Kulturmedium | Apoptose [%] |
|---|---|
| frisch | $1,6 \pm 0,2$ |
| konditioniert | $5,1 \pm 0,2$ |
| konditioniert + monoklon. anti-$\alpha_V$ | $0,5 \pm 0,3$ |
| konditioniert + monoklon. anti-IAP | $0,3 \pm 0,2$ |

BEISPIEL 11

Erzeugung von mechanischer Spannung durch isometrische Kollagenkontraktion in Fibroblastenkulturen

1. Routinekultiviening, zweidimensional:

Kulturmedium:

Kulturmedium für MRC-5-Fibroblasten (s. Beispiel 1)

Durchführung:

Die verwendete Zellinie MRC-5 wird in ungelatinierten Zellkulturgefäßen ausgesät. Die anschließende Kultivierung erfolgt im Brutschrank bei 37°C und 5 Vol% $CO_2$ in wasserdampfgesättigter Luftatmosphäre. Ein Wechseln des Kulturmediums erfolgt jeden zweiten bis dritten Tag, und bei erreichter Konfluenz werden die Zellen mit einer Teilungsrate von 1:5 bis 1:10 passagiert.

2. Kultivierung in Kollagenmatrizes, dreidimensional:

Die Fibroblasten werden zunächst unter statischen Bedingungen bis zum Erreichen der Konfluenz in 75-$cm^2$-Zellkulturflaschen kultiviert. Die Zellernte erfolgt unter Verwendung von 0,05 % (w/v) Trypsin / 0,02 % (w/v) EDTA in PBS.

2A. Herstellung des Kollagengeles (3 mg / ml):

Materialien:

Vitrogen "100" Kollagen, 4°C, von COHESION Technologies, INC., Palo Alto, CA., USA

10-fach konzentrierte PBS-Stammlösung, pH = 7,4, mit 0,005 mg / ml Phenolrot

0,1 M HCl

0,1 M NaOH

Durchführung:

Das Gesamtvolumen beträgt 2 ml je Ansatz: zu 1,6 ml Vitrogen "100" werden 0,2 ml 10-fach PBS und 0,2 ml 0,1 M NaOH hinzugegeben und der pH Wert auf 7,4 mit 0,1 M HCl bzw. 0,1 M NaOH eingestellt.

2B. Zellaussaat in den hergestellten Kollagengelen:

Es werden $2 \times 10^5$ Zellen je Kollagenmatrix ausgesät. Hierfür werden 100 µl der Zellsuspension (der entsprechende Zelltiter wird nach der Trypsinierung in serumfreiem DMEM eingestellt) in auf 37°C vortemperierte 1,5 ml Reaktionsgefäße vorgelegt, 100 µl der neutralisierten Kollagenlösung hinzugefügt und die resultierende Lösung für 4 min bei 37"C inkubiert. Die Kollagenkonzentration beträgt 1,5 mg / ml. Die Aussaat der Zell- / Kollagensuspension erfolgt in 24-Lochzellkulturplatten oder in 3,5 cm Kulturschalen. Die Polymerisation der Matrix erfolgt bei 37°C für 60 min.

2C. Erzeugung von mechanischer Spannung:

Material:

DMEM-Basalmedium (Dulbecco's Modifiziertes Eagel Medium

Ascorbinsäure-Stammlösung (100fach)
5 mg/ml Ascorbinsäure werden in DMEM-Basalmedium gelöst und sterilfiltriet

Kulturmedium(Spannung) (pro 100 ml)
89 ml DMEM-Basalmedium
10 ml FCS
1 ml Ascorbinsäurestammlösung

Kulturmedium DMEM (pro 100 ml)
90 ml DMEM-Basalmedium
10 ml FCS

Durchführung:

Die Matrizes werden mit Kulturmedium (DMEM + 10 % FCS + 50 µg / ml Ascorbinsäure) überschichtet und im Brutschrank bei 37°C und 8,5 Vol% $CO_2$ in wasserdampfgesättigter Luftatmosphäre für 24 h kultiviert.
Die Zugabe von Ascorbinsäure führt zu einer "isometrischen" Kontraktion der Kollagenmatrix, wodurch die mechanische Spannung auf die Zellen erzeugt wird.
Nach 24 h erfolgt ein Wechsel des Mediums. Die weitere Kultivierung erfolgt mit DMEM + 10 % FCS (s.o.).

Kultivierung der Zellen unter mechanischer Spannung:

Die Matrix haftet für den jeweils angegebenen Zeitraum weiterhin auf dem Boden des Kulturgefäßes.

Kultivierung der Zellen unter entspannten Bedingungen:

Die anhaftende Matrix wird vorsichtig mit einem Spatel vom Untergrund gelöst und "schwimmt" ohne Kontakt zum Untergrund im Kulturmedium. Die Spannung wird aufgehoben. Die Kultivierung erfolgt für den jeweils angegebenen Zeitraum.

BEISPIEL 12

Einfluß mechanischer Spannung auf die Apoptose von MRC-5 Fibroblasten:

Durchführung:

Die Zellen werden wie in Beispiel 11 angegeben kultiviert. Tag "0" entspricht dem Zeitpunkt nach 24 h Kultivierung mit Ascorbinsäure-haltigem Medium vor der weiteren Kultivierung unter ge- bzw. entspannten Bedingungen. Nach den genannten Zeiten erfolgt die Fixierung der Zell- / Kollagenmatrix in 4 % (w/v) Paraformaldehyd in PBS und anschließende DAPI-Färbung. Die erhaltenen Ergebnisse sind in der folgenden Tabelle aufgeführt.

| Kultivierung | Kultivierungsdauer | Apoptose [%] |
|---|---|---|
| Vorkultivierung | Tag "Null", 0 h | 1,3±0,2 |
| entspannt | 24 h | 10,2±0,7 |
| entspannt | 48 h | 11,5±0,5 |
| entspannt | 72 h | 11,2±0,6 |
| gespannt | 24 h | 2,4±0,2 |
| gespannt | 48 h | 1,7±0,2 |
| gespannt | 72 h | 1,8±0,3 |

BEISPIEL 13

Einfluß von Thrombospondin-1, von anti-Tsp1 und anti-Rezeptorantikörpern auf die spannungsabhängige Apoptose von MRC-5-Fibroblasten:

Verwendete Substanzen und Antikörper:

Substanzen:

1. gereinigtes Tsp1, von Dr. Vischer, Münster, Deutschland, 20 µg/ml.

2. Chondramid A, ein aus Myxobakterien isoliertes Cytostatikum, von Prof. H. Reichenbach, GBF, Braunschweig, Deutschland, 1 µM.

Spezifische Antikörper:

1. monoklonaler Antikörper aus Maus gegen humanes CD47, von Cymbus Biotechnology LTD, UK, 50 µg/ml (siehe Beispiel 1)

2. monoklonaler und polyklonaler Antikörper gegen Tsp1, von Dr. Vischer, Münster, Deutschland, 50 µg /ml.

3. monoklonaler Antikörper gegen die Integrinuntereinheit β3, Chemicon International Inc., Kanada, Katalog-Nr.: MAB1957, 50 µg/ml.

Durchführung:

Die Vorkultivierung einschließlich der Erzeugung der mechanischen Spannung erfolgt wie in Beispiel 11 beschrieben. "0 h" entspricht dem Zeitpunkt nach 24 h Kultivierung mit Ascorbinsäure-haltigem Medium vor der weiteren Kultivierung unter ge- bzw. entspannten Bedingungen und Zugabe der genannten Substanzen. Die weitere Kultivierungszeit beträgt 24 h. Die erhaltenen Ergebnisse sind in der folgenden Tabelle aufgeführt.

| Kultivierung | Kulturmedium | Zeitraum | Apoptose [%] |
|---|---|---|---|
| Vorkultivierung | DMEM / FCS / Ascorbinsäure | Tag "Null", 0 h | $1,1 \pm 0,2$ |
| gespannt | DMEM / FCS | 24 h | $1,0 \pm 0,2$ |
| entspannt | DMEM/FCS | 24 h | $6,2 \pm 0,2$ |
| entspannt | DMEM/FCS+ mAk-IAP | 24 h | $0,5 \pm 0,1$ |
| entspannt | DMEM/FCS+ mAk-$\beta$3 | 24 h | $0,9 \pm 0,2$ |
| entspannt | DMEM/FCS+ mAk-Tsp1 | 24 h | $0,5 \pm 0,1$ |
| entspannt | DMEM/FCS+ Tsp1 | 24 h | $10,5 \pm 0,5$ |

BEISPIEL 14

Der Einfluß von Chondramid A auf das Cytoskelett von MRC-5 (Kultivierung in Kollagenmatrizen):

Durchführung:

Die Vorkultivierung und Spannung mit Ascorbinsäure-haltigen Kulturmedium erfolgt wie in Beispiel 11 beschrieben. Bei der das Chondramid enthaltenden Probe wird folgendermaßen vorgegangen: Das Ascorbinsäure-haltige Medium wird entfernt und mit 1 μM Chondramid A in Kulturmedium für 1 h inkubiert. Hiernach erfolgt das Lösen der Matrix vom Untergrund und ein Wechsel des Kulturmediums. Die weitere Kulivierungsdauer sämtlicher Ansätze beträgt 8 h. Die erhaltenen Ergebnisse sind in der folgenden Tabelle aufgeführt.

| Kultivierung | Kulturmedium | Zeitraum | Apoptose (%) |
|---|---|---|---|
| gespannt | DMEM / FCS | 8 h | $0,9 \pm 0,2$ |
| entspannt | DMEM / FCS | 8 h | $2,7 \pm 0,2$ |
| entspannt | DMEM / FCS + Chondramid A | 8 h | $1,0 \pm 0,2$ |

BEISPIEL 15

Immunchemischer Nachweis von IAP und der Integrinuntereinheit $\beta_3$ auf MRC-5 Fibroblasten unter verschiedenen Kulturbedingungen:

Durchführung:

Die Vorkultivierung erfolgt wie in Beispiel 11 (Routinekultivierung, zweidimensional:) beschrieben. Die weitere Kultivierung unter ge- bzw. entspannten Bedingungen erfolgt für 72 bis 96 h.

Immunfärbung:

- Entfernung des Kulturmediums

- Zell- / Kollagenmatrix 1 x mit PBS (37°C) waschen

- 30 min 2 % (w/v) Paraformaldehyd in PBS bei 4°C fixieren
  Die folgenden Arbeitsschritte erfolgen auf einem Heidolph Duomax Plattenschüttler (Stufe 2) bei Raumtemperatur.

- 2x für jeweils 5 min in PBS

- 1 h in 0,5 % (vlv) Tween-20 + 0,5 % (w/v) BSA in PBS absättigen

- 2x für jeweils 5 min in 0,5 % (viv) Tween-20 in PBS waschen

- 3h Inkubation mit monoklonalem Maus-anti-humanes CD47 (Cymbus Biotechnology LTD, UK, s. Beispiel 1) bzw. monoklonalem Maus-anti-humanes β3 Antikörper (Chemicon International Inc., Kanada, Katalog-Nr.: MAB1957), 1:100 in 0,5 % (v/v) Tween-20 in PBS

- 3x für jeweils 5 min in 0,5 % (v/v) Tween-20 in PBS waschen

Die folgenden Arbeitsschritte erfolgen lichtgeschützt:

- 2 h Inkubation mit Cy3™ konjugiertem F(ab)2 Fragment aus Kaninchen gegen Maus IgG, (Dianova, Hamburg, Deutschland), 1:200 in 0,5 % (v/v) Tween-20 in PBS waschen

- 3x für jeweils 5 min in 0,5 % (v/v) Tween-20 in PBS waschen

- Kollagenmatrix mit PBS überschichten

- Fluoreszenzmikroskopische Auswertung (Abb. 40-fach)

Anmerkung: die Negativkontrolle wird auf zwei Arten durchgeführt:

a) anstelle des spezifischen CD47 Antikörpers wird unspezifisches Maus IgG in der gleichen Konzentration verwendet.

b) der Zellrasen wird nur mit Cy3™ (Dianova, Hamburg, Deutschland) konjugierten F(ab)$_2$ Fragment inkubiert.

Alle gefärbten Zellen waren für das Integrin positiv. IAP konnte nur auf den entspannt kultivierten Zellen nachgewiesen werden. Die gespannt kultivierten Zellen zeigen keine IAP Expression.

BEISPIEL 16

Bestimmung des Einflusses von Dehnung und Stauchung der Zellen auf den Apoptoseindex:

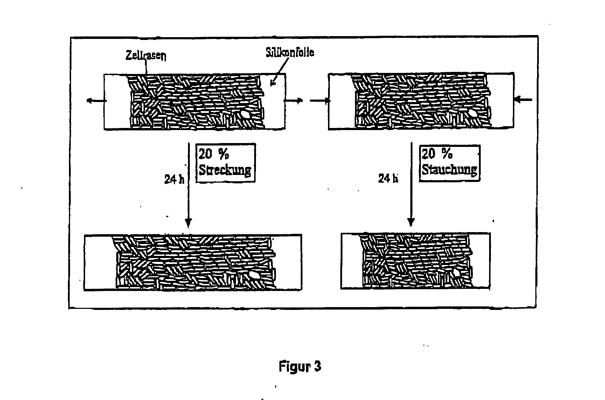

Um den Einfluss von mechanischen Kräften, die aus der Dehnung der Zellen resultieren, untersuchen zu können, werden Zellen auf Silikonfolien ausgesät. Einen Tag nach Erreichen der Konfluenz wird die Folie in die Streckapparatur eingebaut und um 20 % gedehnt. Nach 24 beziehungsweise 48 h Inkubation unter Kulturbedingungen wird der Apoptoseindex bestimmt. Zur Stauchung der Zellen findet die Aussaat auf einer bereits vorgespannten Folie statt. Diese Folie wird einen Tag nach Erreichen der Konfluenz um 20 % entspannt. Dies ist schematisch in Figur 3 gezeigt. Wiederum wird nach 24 beziehungsweise 48 h der Apoptoseindex bestimmt.

BEISPIEL 17

Bestimmung des Einflusses von Dehnung der Zellen auf den Proliferatons- und Apoptoseindex

A) Messen des Proliferationsindex;

Zur Quantifizierung der in einem Experiment erhaltenen Anzahl proliferierenden Zellen wird die Proliferationsrate verwendet. Diese ist folgendermaßen definiert:

$$PR\ [\%] = \frac{Z_{prolif}}{Z} \cdot 100$$

wobei PR= Proliferationsrate [%]
$Z_{prolif}$ = Anzahl der proliferierenden Zellen
Z = Gesamtzellzahl.

Zur Ermittlung der Proliferationsrate werden mit Hilfe des Computerprogrammes "ZellCount" in drei unabhängig voneinander durchgeführten Experimenten jeweils so viele Gesichtsfelder ausgezählt, bis eine Gesamtzellzahl von 1000 Zellen erreicht wird. Die Einstellung des Hintergrundes wird so gewählt, dass sowohl die lebenden als auch die apoptotischen Zellen gezählt werden.

Nachweis proliferierender Zellen durch BrdU-Markierung:

| Lösungen: | Proteine: |
|---|---|
| •**PBS** <br><br> •**70 % Ethanol** <br><br> •**3 N HCl** <br><br> •**BrdU/dC-Lösung** <br> 15 mM BrdU + 15 mM dC in Milli-Q, bei -20°C lagern. Arbeitsverdünnung: 1 : 1000 in Kulturmedium <br><br> •**PBT** <br> PBS + 0,5 % (v/v) Tween-20 <br><br> •**PBT-BSA** <br> PBT + 0,5 % (w/v) BSA <br><br> •**DAPI-Lösung** <br> 30 nM DAPI in Methanol | •**MAK-BrdU** <br> Monoklonales Maus-anti-BrdU-IgG Konzentration der Rohfraktionen: 20-250 µg/ml <br> Arbeitskonzentration: 0,25 µg/ml in PBT-BSA <br><br> •**Maus IgG** <br> Gesamtproteingehalt: 13,8 mg/ml wird nach dem Ansetzen in PBT-BSA sterilfiltriert <br> Arbeitskonzentration: 25 µg/ml in PBT-BSA <br><br><br><br> •**BT-KAM** <br> biotinyliertes-Kaninchen-anti-Maus-IgG <br> Gesamtproteingehalt: 1,0 mg/ml <br> Arbeitsverdünnung: 1 : 3000 in PBT-BSA <br><br> •**Cy3™-SP** <br> Cy3™ konjugiertes Streptavidin <br> Konzentration: 1,8 mg/ml <br> Arbeitskonz.: 5 µg/ml in PBT-BSA <br> Auswertung: Nikon Filterblock G. <br> Anregungswellenlänge: 553 nm. <br> Emissionswellenlänge: 575 mn. |

Durchführung:

Der Zellrasen wird für 30 min mit 0,2 ml/cm² der 1:1000 in Kulturmedium verdünnten BrdU/dC-Lösung inkubiert. Nach erfolgter Inkubation wird zweimal mit PBS gewaschen, der Zellrasen mit 0,2 ml/cm² 70% EtOH für 30 min bei 4°C fixiert und dreimal mit PBS gewaschen.

Die nun folgenden Schritte werden unter leichtem Schwenken (Duomax 1030, Stufe 2) bei Raumtemperatur durchgeführt:

Die Denaturierung der DNS erfolgt durch Zugabe von 0,2 ml/cm² 3 N HCl für 20 min, worauf fünfmal mit PBS gewaschen wird. Es werden 0,2 ml/cm² des MAK-BrdU zugegeben und 30 min geschwenkt. Nach erfolgter Markierung wird fünfmal mit PBT gewaschen, 0,2 ml/cm² BT-Kam hinzugegeben, für 3 Stunden inkubiert und weitere fünfmal für 3 Minuten mit PBT gewaschen. Die Färbung erfolgt durch Zugabe von 0.2 ml/cm² Cy3-SP für eine Stunde und wird durch fünfmaliges Waschen mit PBT für jeweils 3 Minuten beendet. Zur Bestimmung der Proliferationsrate muss eine DAPI Gegenfärbung durchgeführt werden und die Präparate nach Überschichten des Zellrasens mit PBS unter dem Fluoreszenzmikroskop ausgewertet werden.

B) Kultivierung der Zellen:

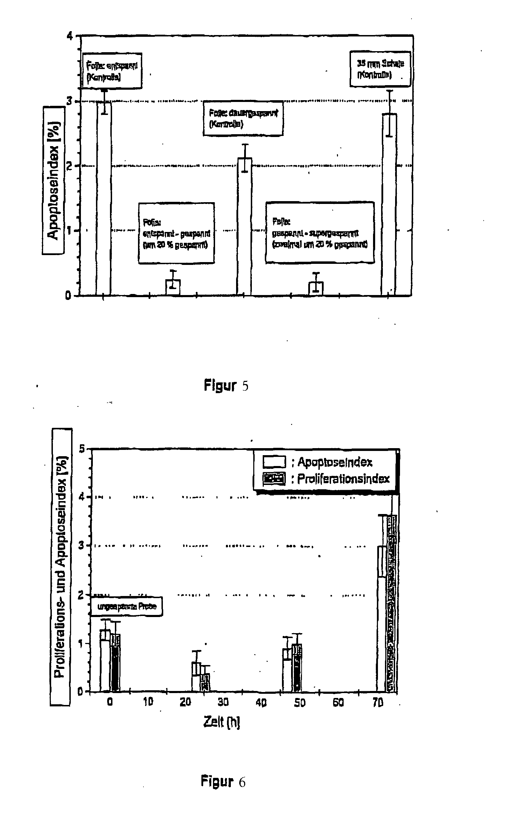

Um den Einfluss von mechanischen Kräften auf die Apoptose- und Proliferationsrate untersuchen zu können, werden Zellen auf Silikonfolien ausgesät. Einen Tag nach Erreichen der Konfluenz werden die Folien in die Streckapparatur eingebaut und um 20 % gedehnt. Nach 24, 48 und 72 h Inkubation unter Kultivierungsbedingungen wird der Apoptose- und Proliferationsindex bestimmt. Zur Kontrolle dienen Zellen, die auf ungespannten Silikonfolien über den gleichen Zeitraum hinweg kultiviert wurden. Die erhaltenen Ergebnisse sind in Figur 6 dargestellt. Bei den gespannten Folien liegen sowohl der Proliferationsindex als auch der Apoptoseindex über einen Zeitraum von 2 Tagen deutlich unter den Werten eines 24 h postkonfluenten Zellrasens (vgl. Figur 6). Dies lässt darauf schließen, dass sich die apoptotischen Zellen nicht einfach von der Siliconfolie ablösen und in den Ergebnissen bei der Bestimmung des Apoptoseindexes nicht berücksichtigt werden können. Wäre dies der Fall gewesen, so hätte dies einen wesentlich höheren Proliferationsindex zur Folge gehabt 72 h nach dem Spannen der Sillkonfolien ist ein rascher Anstieg des Apoptoseindexes und der Proliferationsrate zu verzeichnen, was darauf hin deutet, dass der Reiz, welcher durch das Spannen induziert wird, nach diesem Zeitraum keine Wirkung mehr zeigt, beziehungsweise die Zelle relaxiert ist und das Cytoskelett nicht mehr in gespanntem Zustand vorliegt.

Bei den statisch kultivierten Fibroblasten ist ein Konstanter Anstleg des Apoptose- und Proliferationsindexes zu erkennen.

BEISPIEL 18

Untersuchung der Expression der $\beta_3$-Untereinheit des Integrins $\alpha_v\beta_3$ und des IAP unter ge- beziehungsweise entspannten Kulturbedingungen:

Einen Tag nach dem Spannen der Silikonfolien werden die MRC-5 fixiert und mit Hilfe der indirekten Immunfluoreszenzfärbung gefärbt, um die Expression der $\beta_3$-Untereinheit des Integrins $\alpha_v\beta_3$ und des IAP charakterisieren zu können. Die Ergebnisse der Färbungen zeigen, daß das IAP auf Fibroblasten, welche um 20 % gestreckt wurden, schwächer exprimiert wird als bei Zellen, die auf der ungespannten Siliconfolie kultiviert wurden. Dieses Ergebnis deckt sich mit den zuvor ermittelten Apoptose- und Proliferationsindices.

BEISPIEL 19

Verwendung von Antikörpern oder Peptiden als aktive Wirksubstanzen in pharmazeutischen Formulierungen:

Die identifizierten anti-apoptotisch wirksamen Verbindungen könnten als aktive Wirksubstanzen in pharmazeutischen Formulierungen zur Behandlung von Wunderkrenkungen eingesetzt werden.

Beispielsweise werden dazu Antikörper zweckmäßigerweise in einer Konzentration von 3-5 mg pro ml in folgender Formulierung eingesetzt:

- Wasser für Injektionszwecke

- Polysorbat 80

- Dinatriumhydragenphosphat/Natriumdihydrogenphosphat

- Natriumchlorid

Diese Formulierung wird als Sprühlösung verabreicht (aufgesprüht),

BEISPIEL 20

Inkubation von Fibroblasten mit anti-apoptotisch wirksamen Peptiden

Die Zellen werden unter statischen Kulturbedingungen wie in Beispiel 1/Variante 1B1 kultiviert. Die Zellen werden in die entsprechenden Kulturgefäße (z. B. 24-Lochplatte/0,5 ml pro Kavität) ausgesät und 3 Tage nach dem Erreichen der vollständigen Konfluenz für den Test eingesetzt. Die Zellen werden mit neuem Medium versorgt:

(a) frisches Kulturmedium [Basisrate der Apoptose]

(b) frisches Kulturmedium mit 1 $\mu$g/ml TSP-1 [Apoptose-induzierende Substanz; Kontrolle]

(c) Kulturmedium (b) + Peptid der SEQ ID NO 1; 1 mM

(d) Kulturmedium (b) + Peptid der SEQ ID NO 2; 1 mM

(e) Kulturmedium (b) + Peptid der SEQ ID NO 3; 1 mM

(f) Kulturmedium (b) + Peptid der SEQ ID NO 4; 1 mM

(g) Kulturmedium (b) + Peptid der SEQ ID NO 7; 1 mM

(h) Kulturmedium (b) + Peptid der SEQ ID NO 8; 1 mM

(i) Kulturmedium (b) + Peptid der SEQ ID NO 9; 1 mM

(j) Kulturmedium (b) + Peptid der SEQ ID NO 10; 1 mM

(k) Kulturmedium (b) + Peptid der SEQ ID NO 11; 1 mM

(l) Kulturmedium (b) + Peptid der SEQ ID NO 5; 1 mM

(m) Kulturmedium (b) + Peptid der SEQ ID NO 6; 1 mM

Nach 24 h Inkubation unter Kulturbedingungen (Beispiel 1) werden die Zellen fixiert, mit DAPI gefärbt und morphologisch unter dem Fluoreszenzmikroskop untersucht. Die apoptotischen Zellen und die Gesamtzellzahl werden bestimmt und der Apoptoseindex berechnet. (Prozent apoptotischer Zellen). Die Daten von 3 unabhängigen Experimenten mit der Angabe der Mittelwerte und der Standardabweichung sind in der folgendenTabelle angegeben.

Es werden folgende Peptide getestet:

| SEQ ID NO | Aminosäure-Sequenz | Apoptose-Index [%] | Inhibitions-Index [%] |
|---|---|---|---|
| K | Kontrolle | 4,23 ± 0,23 | |
| (1) | R-A-Y-V-V-M | 1,21 ± 0,29 | 71,4 ± 6,8 |
| (2) | R-W-Y-V-V-M | 2,00 ± 0,29 | 53,7 ± 6,8 |
| (3) | R-Y-Y-V-V-M | 1,45 ± 0,04 | 65,3 ± 0,9 |
| (4) | R-E-Y-V-V-M | 0,87 ± 0,32 | 80,4 ± 7,0 |
| (7) | R-G-Y-V-V-M | 2,00 ± 0,59 | 53,7 ± 13,9 |
| (8) | R-M-Y-V-V-M | 2,46 ± 0,29 | 42,8 ± 6,9 |
| (9) | R-T-Y-V-V-M | 3,19 ± 0,50 | 25,4 ± 11,8 |
| (10) | R-N-Y-V-V-M | 3,70 ± 1,11 | 13,5 ± 16,2 |
| (11) | R-D-Y-V-V-M | 2,66 ± 0,71 | 38,9 ± 16,7 |
| (5) | K-R-A-Y-V-V-M-W-K-K | 0,42 ± 0,21 | 90,1 ± 4,9 |
| (6) | K-R-E-Y-V-V-M-W-K-K | 0,24 ± 0,18 | 94,4 ± 4,2 |
| K: Kein Peptid eingesetzt. | | | |

Man erkennt deutlich die Apooptose-inhibierende Wirkung dieser Peptide, die diese für die erfindungsgemäße Verwendung zur Behandlung von Wunden geeignet macht.

Der Inhibitionsindex der eingesetzten Peptide wird wie folgt errechnet:

$$\text{Inhibitionsindex [\%]} = 100 - (\text{gemessener Apoptose-Index} * 100 /$$

$$\text{Apoptose-Index Kontrolle})$$

## Patentansprüche

1. Verwendung von Substanzen zur Herstellung eines Medikamentes zur Förderung der Wundheilung, wobei diese Substanzen entweder an IAP und/oder an Integrin $\alpha_v\beta_3$ und/oder an Thrombospondin 1 so binden, daß die Bindung zwischen Thrombospondin 1 und IAP und/oder Integrin $\alpha_v\beta_3$ und die durch Kernfragmentierung gekennzeichnete Apoptose von Zellen inhibiert wird, und wobei diese Substanzen ausgewählt sind aus der Gruppe bestehend aus:

(i) spezifische Antikörper oder funktionelle Varianten hiervon gegen Thrombospondin 1, IAP und/oder Integrin $\alpha_v\beta_3$ ist, oder

(ii) Peptide, ausgewählt aus den in den SEQ ID NOs 1 bis 11 dargestellten Aminosäuresequenzen bzw. davon abgeleiteten Peptidmimetika oder diese Sequenzen umfassende Proteine.

**2.** Verwendung nach Anspruch 1, **dadurch gekennzeichnet, daß** zunächst ein Idendfikationsverfahren der Stufen (i) bis (v) durchgeführt wird, wobei man

**(i)** Fibroblasten züchtet, die sowohl IAP als auch das Integrin $\alpha_v\beta_3$ exprimieren,

**(ii)** die Zellen zur Bildung eines Apoptose-induzierenden Stoffes veranlaßt, und/oder einen Stoff bzw. Stoffe zusetzt, der (die) Apoptose induziert/induzieren,

**(iii)** die Substanz zusetzt,

**(iv)** die Apoptoserate misst, und

**(v)** Substanzen, die eine verringerte Apoptoserate hervorbringen, auswählt, und dann die so identifizierte Substanz (das Identifikat) mit einem pharmazeutisch annehmbaren Träger mischt.

**3.** Verwendung nach Anspruch 2, wobei die Substanzen entweder an IAP und/oder an Integrin $\alpha_v\beta_3$ auf Fibroblasten und/oder an Thrombospondin 1 so binden, daß die Bindung zwischen Thrombospondin 1 und dem IAP und/oder dem Integrin $\alpha_v\beta_3$ inhibiert und die Apoptoserate der Zellen um mehr als 10% verringert wird.

## Claims

**1.** Use of substances for producing a medicament for promoting the healing of wounds, wherein said substances bind either to IAP and/or to integrin $\alpha_v\beta_3$ and/or to thrombospondin 1 in such way that the binding between thrombospondin I and IAP and/or integrin $\alpha_v\beta_3$, and the apoptosis of cells **characterised by** fragmentation of the nucleus, are inhibited, and wherein said substances are selected from the group comprising:

(i) specific antibodies, or functional variants thereof, against thrombospondin 1, IAP and/or integrin $\alpha_v\beta_3$, or

(ii) peptides selected from the amino acid sequences represented in the SEQ ID Nos. 1 to 11, or peptide mimetics derived therefrom, or proteins comprising these sequences.

**2.** Use according to claim 1, **characterised in that** a method of identification of stages (i) to (v) is first performed, wherein

(i) fibroblasts which express both IAP and integrin $\alpha_v\beta_3$ are cultivated,

(ii) the cells are caused to form an apoptosis-inducing material, and/or a material or materials which induces/ induce apoptosis is/are added,

(iii) the substance is added.

(iv) the rate of apoptosis is measured, and

(v) substances which produce a reduced rate of apoptosis are selected and the substance which has been identified in this way (the identificate) is then mixed with a pharmaceutically acceptable vehicle.

**3.** Use according to claim 2, wherein the substances bind either to IAP and/or to integrin $\alpha_v\beta_3$ on fibroblasts and/or to thrombospondin 1 in such a way that the binding between thrombospondin 1 and IAP and/or integrin $\alpha_v\beta_3$ is inhibited and the rate of apoptosis of the cells is reduced by more than 10%.

## Revendications

**1.** Utilisation de substances pour la préparation d'un médicament destiné à accélérer la cicatrisation de blessures, ces substances se liant à l'IAP et/ou à l'intégrine $\alpha_v\beta_3$ et/ou à la thrombospondine 1 de façon à inhiber la liaison entre la thrombospondine 1 et l'IAP et/ou l'intégrine $\alpha_v\beta_3$ et l'apoptose des cellules **caractérisée par** une fragmentation du noyau, et ces substances étant choisies dans le groupe constitué par

(i) des anticorps spécifiques ou leurs variants fonctionnels, dirigés contre la thrombospondine 1, l'IAP et/ou l'intégrine $\alpha_v\beta_3$, ou

(ii) des peptides choisis parmi les séquences d'aminoacides représentées par SEQ ID NO. 1 à 11 ou des peptides mimétiques qui en dérivent ou des protéines comprenant ces séquences.

2. Utilisation selon la revendication 1, **caractérisée en ce que** l'on effectue d'abord un procédé d'identification comportant les étapes (i) à (v), dans lesquelles

(i) on cultive des fibroblastes qui expriment aussi bien l'IAP que l'intégrine $\alpha_v\beta_3$,

(ii) on amène les cellules à former un produit qui induit l'apoptose, et/ou on ajoute un ou plusieurs produits qui induisent l'apoptose,

(iii) on ajoute la substance,

(iv) on mesure le taux d'apoptose, et

(v) on sélectionne des substances qui provoquent un taux d'apoptose réduit, puis on mélange la substance ainsi identifiée (substance identifiée) avec un support pharmaceutiquement acceptable.

3. Utilisation selon la revendication 2, dans laquelle les substances se lient à l'IAP et/ou à l'intégrine $\alpha_v\beta_3$ sur des fibroblastes et/ou à la thrombospondine 1 de façon à inhiber la liaison entre la thrombospondine 1 et l'IAP et/ou l'intégrine $\alpha_v\beta_3$ et à réduire le taux d'apoptose des cellules de plus de 10 %.

(3)

(2)

(4)

(5) →

**Figur 1**

(1)

**Figur 2**

Figur 3

**Figur** 4

**Figur** 5

**Figur** 6

SEQUENZ PROTOKOLL

<110> CYTOTOOLS GMBH

<120> MITTEL, DIE DIE APOPTOSE BEI AN DER WUNDHEILUNG BETEILIGTEN ZELLEN INHIBIEREN

<130> C 679 WO

<150> DE 101 09 136.2
<151> 2001-02-26

<160> 11

<170> PATENTIN VERSION 3.1

<210> 1
<211> 6
<212> PRT
<213> KÜNSTLICHE SEQUENZ

<220>
<223> SYNTHETISCHES PEPTID

<400> 1

```
                              ARG ALA TYR VAL VAL MET
                              1               5
```

<210> 2
<211> 6
<212> PRT
<213> KÜNSTLICHE SEQUENZ

<220>
<223> SYNTHETISCHES PEPTID

<400> 2

```
                              ARG TRP TYR VAL VAL MET
                              1               5
```

<210> 3
<211> 6
<212> PRT
<213> KÜNSTLICHE SEQUENZ

<220>
<223> SYNTHETISCHES PEPTID

<400> 3

```
                              ARG TYR TYR VAL VAL MET
                              1               5
```

<210> 4
<211> 6
<212> PRT
<213> KÜNSTLICHE SEQUENZ

<220>
<223> SYNTHETISCHES PEPTID

<400> 4

```
ARG GLU TYR VAL VAL MET
1               5
```

<210> 5
<211> 10
<212> PRT
<213> KÜNSTLICHE SEQUENZ

<220>
<223> SYNTHETISCHES PEPTID

<400> 5

```
LYS ARG ALA TYR VAL VAL MET TRP LYS LYS
1               5                   10
```

<210> 6
<211> 10
<212> PRT
<213> KÜNSTLICHE SEQUENZ

<220>
<223> SYNTHETISCHES PEPTID

<400> 6

```
LYS ARG GLU TYR VAL VAL MET TRP LYS LYS
1               5                   10
```

<210> 7
<211> 6
<212> PRT
<213> KÜNSTLICHE SEQUENZ

<220>
<223> SYNTHETISCHES PEPTID

<400> 7

```
ARG GLY TYR VAL VAL MET
1               5
```

<210> 8
<211> 6
<212> PRT
<213> KÜNSTLICHE SEQUENZ

<220>
<223> SYNTHETISCHES PEPTID

<400> 8

```
ARG MET TYR VAL VAL MET
1               5
```

<210> 9
<211> 6
<212> PRT
<213> KÜNSTLICHE SEQUENZ

<220>
<223> SYNTHETISCHES PEPTID

<400> 9

```
ARG THR TYR VAL VAL MET
1               5
```

<210> 10
<211> 6
<212> PRT
<213> KÜNSTLICHE SEQUENZ

<220>
<223> SYNTHETISCHES PEPTID

<400> 10

```
ARG ASN TYR VAL VAL MET
1               5
```

<210> 11
<211> 6
<212> PRT
<213> KÜNSTLICHE SEQUENZ

<220>
<223> SYNTHETISCHES PEPTID

<400> 11

```
ARG ASP TYR VAL VAL MET
1               5
```